# EUROPEAN PATENT APPLICATION

(11) **EP 2 620 142 A1**
(43) Date of publication of application: **31.07.2013**
(21) Application number: 12152943.2
(22) Date of filing: 27.01.2012
(51) Int. Cl.: A61K 31/00, A61K 31/4355, A61P 3/08

(54) **Hedgehog signaling pathway involved in energy metabolism**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner

(57) **Abstract**

The present invention relates to a modulator of the Smoothened (Smo) receptor capable of altering cellular glucose uptake for use in the treatment of a disease, wherein (a) the modulator is an activator of the Smoothened (Smo) receptor capable of increasing cellular glucose uptake and is for use in the treatment of diabetes, glucose intolerance, hyperglycemia, obesity, overweight, sepsis and diseases associated therewith; or (b) wherein the modulator is an inhibitor of the Smoothened (Smo) receptor capable of decreasing cellular glucose uptake and is for use in the treatment of hypoglycaemia. Further, the invention relates to a method of altering the glucose uptake, the lactate level, the NAD⁺/NADH ratio and/or the NADP⁺/NADPH ratio of a cell and a method of identifying a modulator of the Smoothened (Smo) receptor capable of altering the glucose uptake, the lactate level, the NAD⁺/NADH ratio and/or the NADP⁺/NADPH ratio of a cell. Also, the invention relates to a method of increasing uptake of glucose or a glucose analogue labelled with a detectable moiety.

## Description

The present invention relates to a modulator of the Smoothened (Smo) receptor capable of altering cellular glucose uptake for use in the treatment of a disease, wherein (a) the modulator is an activator of the Smoothened (Smo) receptor capable of increasing cellular glucose uptake and is for use in the treatment of diabetes, glucose intolerance, hyperglycemia, obesity, overweight, sepsis and diseases associated therewith; or (b) wherein the modulator is an inhibitor of the Smoothened (Smo) receptor capable of decreasing cellular glucose uptake and is for use in the treatment of hypoglycaemia. Further, the invention relates to a method of altering the glucose uptake, the lactate level, the NAD⁺/NADH ratio and/or the NADP⁺/NADPH ratio of a cell and a method of identifying a modulator of the Smoothened (Smo) receptor capable of altering the glucose uptake, the lactate level, the NAD⁺/NADH ratio and/or the NADP⁺/NADPH ratio of a cell. Also, the invention relates to a method of increasing uptake of glucose or a glucose analogue labelled with a detectable moiety.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Diabetes, obesity and cancer affect upwards of 15% of the world's population. Interestingly, all three of these diseases juxtapose dysregulation of intracellular signalling with altered metabolic state. Exactly which factors define stable metabolic setpoints *in vivo* remains ill-defined.

Hedgehog was initially described as a morphogen critical to defining body patterning throughout development¹, In mammals, ligand binding to the inhibitory cell surface receptor Patched (Ptch) permits Smoothened (Smo) translocation to the tip of the cilium and liberates zinc-finger family transcription factors Gli1, Gli2 and Gli3 frock proteasomal degradation and inhibitory tethering by Sufu/Kif7, a cascade which leads activation of select Gli-target genes in the nucleus¹⁻⁷. This is referred to as the canonical Hedgehog pathway herein. Three highly homologous mammalian ligands exist for the single *Drosophila* Hedgehog, namely Desert, Indian and Sonic Hedgehog (Dhh, Ihh and Shh respectively). While the majority of Hedgehog-induced biological effects are thought to result from transcriptional regulation of Gli-target genes, several non-canonical modules have also been reported⁷⁻¹².

Downstream biological effects of Hedgehog activation impact stem cell renewal, lineage decision, cell cycle-regulation, migration, and multiple mitogenic signalling pathways^{1-6,1-2}. Accordingly, mutations in the Hedgehog pathway have been causally implicated in a number of disparate cancers¹²⁻¹⁶. Hedgehog signalling blocks adipogenesis¹⁷⁻¹⁹. This biological effect is sensitive to inflammatory state²⁰ and represents one of the few known signalling nodes capable of differentially regulating brown vs. white adipocyte differentiation^{17,21}. All three factors, inflammation, adipocyte turnover, and adipose sub-type distribution show substantial imbalance in obesity and represent targets for therapeutic intervention²²⁻²⁴. Inhibitors of Hedgehog signalling from at least 7 pharmaceutical companies are currently in Phase I/II clinical trials¹³. Intriguingly, despite their advanced clinical development, a clear universal mechanism underlying Hedgehog inhibitor efficacy is lacking. Specifically, potency of canonical Hedgehog pathway inhibition does not match potency of inhibition of cancer growth²⁵. One proposed mechanism to explain these anomalies has been indirect cancer modulation through paracrine or mesenchyme-tumour interaction^{9,12,25-28}. The discordance however remains an enigma.

The technical problem underlying the present invention was to identify alternative and/or improved means and methods modulate cellular energy metabolism.

The solution to this technical problem is achieved by providing the embodiments characterized in the claims.

Accordingly, the present invention relates in a first embodiment to a modulator of the Smoothened (Smo) receptor capable of altering cellular glucose uptake for use in the treatment of a disease, wherein (a) the modulator is an activator of the Smoothened (Smo) receptor capable of increasing cellular glucose uptake and is for use in the treatment of diabetes, glucose intolerance, hyperglycemia, obesity, overweight, sepsis and diseases associated therewith; or (b) wherein the modulator is an inhibitor of the Smoothened (Smo) receptor capable of decreasing cellular glucose uptake and is for use in the treatment of hypoglycaemia.

The term "modulator" as used in accordance with the present invention relates to a given compound with the ability of to modulate either directly or indirectly the Smo receptor so as to alter, i.e. increase or decrease, the biological function or activity of the Smoothened (Smo) receptor either directly or indirectly. In other words, a compound "modulating" the Smo receptor alters the biological function or activity of the Smo receptor in that it increases or decreases said biological function or activity. For example, direct modulation may be achievable by binding to the Smo receptor, whereas indirect modulation may be achievable by interacting with molecules that are involved in the regulation of the Smo receptor, such as regulatory molecules upstream of the Smo receptor.

The Smoothened receptor is well known in the art and refers to a G protein coupled receptor protein of the highly conserved "hedgehog" pathway, i.e. referred to as the canonical Hedgehog signaling pathway herein (Ingham, P. W., et al. 2011. Nature Reviews Genetics 12:393-406). The known biological functions of Hedgehog / Smoothened are vast and constantly being expanded. They include numerous developmental processes such as vasculogenesis, osteoblast, adipocyte, cardioblast, and myoblast differentiation, cell fate, organ development as well as effects in the adult including regulation of stem cell renewal and proliferation, neuronal projection and spermatogenesis. The signal initiated by ligand binding to the Patched receptor causes translocation of Smo (Smoothened), a seven transmembrane G-protein coupled receptor, to the cilium where it initiates a cascade of signaling leading to processing and translocation of the Gli transcription factors to the nucleus. This so called canonical pathway is thought to be responsible, primarily through modification of Hedgehog target gene transcription, to mediate the majority of Hedgehog's biological effects. The Smo receptor has been first described in 1996 ((Alcedo J et al, Cell. 1996 Jul 26;86(2):221-32; van den Heuvel M and Ingham PW. Nature. 1996 Aug 8;382(6591 ):547-51)). The complete human protein and mRNA sequence are available in the databases maintained by the National Center for Biotechnology Information (NCBI), 8600 Rockville Pike, Bethesda MD, 20894 USA, under the accession numbers NP_005622.1 (GI:5032099) and NM_005631.4 (GI:300116182), respectively, at the world wide web address: http:l/www.ncbi.nlm.nih.gov/.

The biological function or activity of the Smo receptor known in the art has been described herein above in some detail. The present invention is, however, based on the finding that the Smoothened receptor is also involved in a non-canonical pathway that regulates cellular energy metabolism. Without being bound to a specific scientific theory, it is believed that said non-canonical pathway does not or not essentially depend upon translocation of the Gli factors. Hence, in accordance with the invention the biological function and activity of Smo that is relevant for the invention and to which is referred to herein, is linked to cellular energy metabolism. Said cellular energy metabolism is not dependent on long-term canonical signaling pathway induced transcriptional gene activation. Instead, it is an effect that can, in the case of an activating modulation of the Smo receptor, be observed already within 5 to 10 minutes *in vitro* and *in vivo* within 10 to 20 minutes after contact with said activator. In contrast, the canonical Hedgehog signaling pathway is less rapid and takes several hours to days for changes to take place relating to energy metabolism. The non-canonical pathway, however, shows more rapid effects with regard to energy metabolism, in particular regarding the biological activities and functions of the Smo receptor that relate to the regulation of glucose uptake, of the lactate level, of the NAD⁺/NADH ratio, of the NADP⁺/NADPH ratio, of G-proteins activated via the Smo-receptor, of Amp-activated protein kinase (Ampk) phosphorylation and/or of the opening of Ca²⁺-channels (as detailed below), upon contact of a cell with a modulator that can be determined in less than 3 hrs, preferably less than 1,5 hrs, such as 1 hr, 45 min, and more preferred, less than 40 min, 35 min, and most preferred less than 30 min, such as less than 25 min, less than 20 min, less than 15 min, less than 10 min, less than 5 min, less than 2,5 min, less than 1 min, less than 30 seconds, less than 15 seconds, less than 5 seconds. It is understood, that any of the above mentioned biological activities or functions of the Smo receptor relating to energy metabolism mediated by the non-canonical Hedgehog pathway may occur at different time points, but in any case earlier than changes mediated by the canonical Hedgehog signaling pathway. For example, opening of Ca²⁺ -channels and influx of Ca²⁺ can be observed as early as 1 second after stimulation of the Smo receptor with an activator as defined herein, whereas AMPK phosphorylation may be detected within less than two minutes of said stimulation. As follows from the above, a modulator of the Smo receptor must affect the non-canonical Hedgehog signaling pathway so that the biological function or activity of the Smo receptor is altered to be considered a modulator in accordance with the invention, but may at the same time also alter the canonical Hedgehog signaling pathway, in the same or in a different direction, i.e. activate or inhibit both pathways or activate or activate one and inhibit the other. Specifically, said biological function or activity includes *inter alia* the regulation of cellular glucose uptake, regulation of lactate level, regulation of NAD⁺/NADH ratio and/or the NADP⁺/NADPH ratio of a cell. As a consequence, altering said biological function or activity will result in an altered level of glucose uptake, an altered level of lactate, an altered NAD⁺/NADH ratio and/or an altered NADP⁺/NADPH ratio. Further, said biological function or activity includes the coupling/uncoupling of G-proteins to the Smo-receptor, the phosphorylation of Amp-activated protein kinase (Ampk) and the opening of Ca²⁺-channels as well as the other biological functions or activities associated with the novel non-canonic Smo signalling pathway described herein, in particular the example section. All these functions or activities can be tested for or either using any of a variety of standard methods known in the art or on the basis of the teachings of the examples provided below, optionally in conjunction with further molecular techniques such as RT-PCR or with the teaching of the documents cited herein. For example, cellular glucose uptake can be assessed by determining depletion of glucose from the cell culture medium, by measuring the amount of labeled glucose within cells, and/or by measuring the amount of (fluorescent) glucose biosensors; the lactate level can be determined by colourimetric or fluorometric enzyme assay, the ratio of the metabolites NAD+, NADP+, NADH, and NADPH can be assessed by a colourimetric alcohol dehydrogenase cycling reaction, in which a tetrazolium dye (MTT) is reduced by NADH in the presence of phenazine methosulfate (PMS); the activity (mediated by phosphorylation) of AMPK can be determined using AMPK-AR constructs combined with a FRET assay or determination of phosphorylation of AMPK by anti-phospho AMPK antibodies; the opening of Ca²⁺ channels can be determined by calcium flux measurement; the coupling of G proteins to the Smo receptor can be assessed by measurement of the GPCR effector targets, cAMP, cGMP and D-myo-inositol 1,4,5-trisphosphate (IP3) as well as a number of homogeneous assay methodologies such as Scintillation Proximity Assay (SPA), Fluorescence Polarization (FP) and Enzyme Fragment Complementation (EFC) and binding of [35S]GTPyS.

The term "altering" as used herein in the context of any of the biological functions or activities described herein of the Smo receptor refers to a quantitative and/or qualitative change in any of the biological functions or activities of the Smo receptor as defined herein to be associated with the non-canonical Hedgehog signalling pathway. For example, and with regard to glucose uptake a modulator of the Smo receptor may alter glucose uptake quantitatively by increasing it or decreasing it in comparison to glucose uptake in the absence of said modulator. The same applies also *mutatis mutandis* to the modification of the lactate level, the NAD⁺/NADH ratio and/or the NADP⁺/NADPH ratio of a cell by a modulator in accordance with the invention.

The decrease of said biological function or activity is effected by compounds designated "inhibitors" herein that can modulate the Smo receptor, wherein the increase of said biological function or activity is effected by compounds designated as "activators" herein that can modulate the Smo receptor.

An inhibitor is a compound that reduces or abolishes the biological function or activity of the Smo receptor. An inhibitor may perform any one or more of the following effects in order to reduce or abolish the biological function or activity of the protein to be inhibited, i.e. the Smo receptor: (i) the transcription of the gene encoding the protein to be inhibited is lowered, i.e. the level of mRNA is lowered, (ii) the translation of the mRNA encoding the protein to be inhibited is lowered, (iii) the protein performs its biochemical function with lowered efficiency in the presence of the inhibitor, and (iv) the protein performs its cellular function with lowered efficiency in the presence of the inhibitor.

Compounds falling in class (i) include compounds interfering with the transcriptional machinery and/or its interaction with the promoter of said gene and/or with expression control elements remote from the promoter such as enhancers, so that the transcription of the Smo receptor is lowered. Compounds of class (ii) comprise antisense constructs and constructs for performing RNA interference (e.g. siRNA) well known in the art (see, e.g. Zamore (2001) Nat Struct Biol. 8(9), 746; Tuschl (2001) Chembiochem. 2(4), 239). Compounds of class (iii) interfere with molecular function of the protein to be inhibited, such as receptor signalling activity and activation of downstream target molecules, so that the Smo receptor protein performs its biochemical function with lowered efficiency. Accordingly, active site binding compounds are envisaged. Class (iv) includes compounds which do not necessarily bind directly to target, but still interfere with its function or activity, for example by binding to and/or inhibiting the function or inhibiting expression of members of a pathway which comprises the target, so that the Smo receptor performs its cellular function with lowered efficiency. These members may be either upstream or downstream of the target within said pathway. For example, such compounds may alter, such as decrease, the affinity or rate of binding of a known ligand to the receptor or compete with a ligand for binding to the receptor or displace a ligand bound to the receptor.

An activator is a compound that increases said biological function or activity of the Smo receptor. An activator may perform any one or more of the following effects in order to increase the biological function or activity of the protein to be activated, i.e. the Smo receptor: (i) the transcription of the gene encoding the protein to be activated is increased, i.e. the level of mRNA is increased, (ii) the translation of the mRNA encoding the protein to be activated is increased, (iii) the protein performs its biochemical function with increased efficiency in the presence of the activator, and (iv) the protein performs its cellular function with increased efficiency in the presence of the activator.

Compounds falling in class (i) include compounds interfering with the transcriptional machinery and/or its interaction with the promoter of said gene and/or with expression control elements remote from the promoter such as enhancers, so that the transcription of the Smo receptor is increased. Compounds of class (ii) comprise compounds that reduce the degradation rate of the protein to be activated, increase mRNA concentration (which may be identical with compounds of class (i)), and/or increase ribosome density. Compounds of class (iii) interfere with molecular function of the protein to be activated, such as receptor signalling activity and activation of downstream target molecules, so that the Smo receptor protein performs its biochemical function with increased efficiency. Accordingly, active site binding compounds are envisaged, i.e. compounds that bind to the Smo receptor. Class (iv) includes compounds which do not necessarily bind directly to target, but still interfere with its function or activity, for example by binding to and/or activating the function or increasing the expression of members of a pathway which comprises the target, i.e. the Smo receptor, so that the Smo receptor performs its cellular function with increased efficiency. These members may be either upstream or downstream of the target within said pathway. For example, such compounds may alter, such as increase, the affinity or rate of binding of a known ligand to the receptor or compete with a ligand for binding to the receptor or displace a ligand bound to the receptor. For example, such molecules may be molecules interacting with the Patched (Ptch) receptor as outlined above. Preferably, activators or inhibitors belong to compounds falling under class (iii) and/or (iv), more preferred under class (iii).

The inhibitor or activator, in accordance with the present invention, may in certain embodiments be provided as a proteinaceous compound or as a nucleic acid molecule encoding the inhibitor or activator. For example, the nucleic acid molecule encoding the inhibitor or activator may be incorporated into an expression vector comprising regulatory elements, such as for example specific promoters, and thus can be delivered into a cell. Methods for targeted transfection of cells and suitable vectors are known in the art, see for example Sambrook and Russel ("Molecular Cloning, A Laboratory Manual", Cold Spring Harbor Laboratory, N.Y. (2001)). Incorporation of the nucleic acid molecule encoding the inhibitor or activator into an expression vector allows to either selectively or permanently elevate the level of the encoded inhibitor or activator in any cell or a subset of selected cells of the recipient. Thus, a tissue- and/or time-dependent expression of the inhibitor or activator can be achieved, for example, restricted to muscle cells.

In a preferred embodiment, the inhibitor reduces the biological function or activity of the Smo receptor by at least 50%, preferably by at least 75%, more preferred by at least 90% and even more preferred by at least 95% such as at least 98% or even by 100% in comparison to said biological function or activity in the absence of said inhibitor. The term reduction by at least, for example 75%, refers to a decreased biological function or activity such that the Smo receptor loses 75% of its function or activity and, consequently, has only 25% of the function or activity remaining as compared to the Smo receptor that is not inhibited. In the case of an activator it is preferred that it increases the biological function or activity of the Smo receptor is an increase by at least 10%, preferably at least 20% such as at least 30%, at least 40%, at least 50%, at least 60%, more preferred by at least 70%, such as at least 80%, at least 90%, at least 100%, and even more preferred by at least 150%, at least 200%, at least 500% or even 1000%.

The function of any of the inhibitors referred to in the present invention may be identified and/or verified by using high throughput screening assays (HTS), where possible. High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

The determination of binding of potential inhibitors or activators, e.g., to the Smo receptor, can be effected in, for example, any binding assay, preferably biophysical binding assay, which may be used to identify binding of test molecules prior to performing the functional/activity assay with the inhibitor. Suitable biophysical binding assays are known in the art and comprise fluorescence polarization (FP) assay, fluorescence resonance energy transfer (FRET) assay and surface plasmon resonance (SPR) assay.

In cases where the inhibitor acts by decreasing the expression level or the activator acts by increasing the expression level of the target protein, e.g., the Smo receptor or an activator thereof, the determination of the expression level of the protein can, for example, be carried out on the nucleic acid level or on the amino acid level.

Methods for determining the expression of a protein on the nucleic acid level include, but are not limited to, northern blotting, PCR, RT-PCR or real RT-PCR. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step, e.g. at 55°C for 1 min as well as a final extension step, e.g. at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, it is well known in the art how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT reaction can be performed, for example, in a 20µl reaction mix containing: 4 µl of 5x AMV-RT buffer, 2 µl of oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case. The resulting products are loaded onto an agarose gel and band intensities are compared after staining the nucleic acid molecules with an intercalating dye such as ethidium bromide or SybrGreen. A lower band intensity of the sample treated with the inhibitor as compared to a non-treated sample indicates that the inhibitor successfully inhibits the protein.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in real time RT-PCR experiments is a DNA intercalating dye such as SybrGreen for monitoring the de novo synthesis of double stranded DNA molecules.

Methods for the determination of the expression of a protein on the amino acid level include, but are not limited to, western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognizing the protein of interest. After washing, a second antibody specifically recognizing the first antibody and carrying a readout system such as a fluorescent dye is applied. The amount of the protein of interest is determined by comparing the fluorescence intensity of the protein derived from the sample treated with the inhibitor and the protein derived from a non-treated sample. A lower fluorescence intensity of the protein derived from the sample treated with the inhibitor indicates a successful inhibitor of the protein. Also of use in protein quantification is the Agilent Bioanalyzer technique.

In accordance with the invention, it is understood that a modulator of the Smo receptor acting as defined herein above is valuable in the treatment of various diseases in which any of the biological functions or activities of the Smo receptor identified herein to be associated with the newly discovered non-canonical pathway are deemed to play a role, either causally or non-causally.

An activator of the Smo receptor capable of increasing cellular glucose uptake can be used to treat any form of diabetes. The disease "diabetes" is well-known in the art and also referred to as "diabetes mellitus" subsuming (heterogenic) metabolic diseases characterized by high blood sugar. The disease is a metabolic disease characterized by high blood sugar of the affected individual, because either the body does not produce (enough) insulin or because the cells do not respond to insulin. The term "diabetes" comprises type 1 diabetes, type 2 diabetes and gestational diabetes. Type 1 diabetes (also known as: insulin-dependent diabetes, juvenile diabetes) is characterized by autoimmunity triggered loss of pancreatic beta-cells resulting in insufficient amounts of insulin. Type 2 diabetes is characterized by cellular insulin resistance (also known as: insulin-independent diabetes, adult-onset diabetes) and/or altered, e.g. reduced, insulin secretion. Gestational diabetes describes high blood glucose levels during pregnancy of women that were not diabetic before their pregnancy. The term "diabetes" further comprises Maturity onset diabetes of the young (MODY) that refers to several hereditary forms of diabetes caused by mutations in an autosomal dominant gene (sex independent, i.e. inherited from any of the parents) disrupting insulin production. Also comprised is a condition termed "pre-diabetes" describing the state when a subject's blood glucose levels are higher than normal but not high enough to qualify for a diagnosis of type 2 diabetes. Further comprised is also type 3 diabetes characterized by an insufficient insulin production by the brain, neonatal diabetes, diabetes type 1.5 (also known as latent autoimmune diabetes in adults, ketosis-prone diabetes mellitus type 2) and steroid-induced diabetes as well as any inherited forms of diabetes. It is accepted for most forms of diabetes that a genetic factor (e.g., a mutation) may be involved in the onset and/or maintenance of diabetes. Forms of diabetes involving genetic defects affecting beta-cell function are, e.g., MODY3 (Chromosome 12, HNF-1α), MODY2 (Chromosome 7, glucokinase), MODY1 (Chromosome 20, HNF-4α), MODY4 (also known as IPF-1) (Chromosome 13, insulin promoter factor-1), MODY5 (Chromosome 17, HNF-1β), MODY6 (Chromosome 2, NeuroD1), and forms of diabetes resulting from mutations in the mitochondrial DNA leading to beta-cell dysfunction. Forms of diabetes involving genetic defects affecting insulin action are, e.g., type A insulin resistance, leprechaunism, Rabson-Mendenhall syndrome, and lipoatrophic diabetes. Other genetic syndromes sometimes associated with diabetes are, e.g., Down syndrome, Klinefelter syndrome, Turner syndrome, Wolfram syndrome, Friedreich ataxia, Huntington chorea, Laurence-Moon-Biedl syndrome, myotonic dystrophy, porphyria and Prader-Willi syndrome. Forms of diabetes caused by diseases of the exocrine pancreas include, e.g., pancreatitis, trauma/pancreatectomy, neoplasia, cystic fibrosis, hemochromatosis, and fibrocalculous pancreatopathy. Further forms of diabetes caused by endocrinopathies include, e.g., Acromegaly, Cushing's syndrome, glucagonoma, pheochromocytoma, hyperthyroidism, somatostatinoma, and aldosteronoma. Also, forms of diabetes that are drug- or chemical-induced forms of are included. For example, vacor, pentamidine, nicotinic acid, glucocorticoids, thyroid hormone, diazoxide, β-adrenergic agonists, thiazides, dilantin and γ-Interferon have been causally linked to diabetes. Furthermore, encompassed by the term "diabetes" are forms of diabetes caused by infection, e.g., with rubella (congenital) or cytomegalovirus. Also envisaged is the treatment of hyperglycemias caused by diabetes, drugs (e.g., corticosteroids, octreotide, beta blockers, epinephrine, thiazide diuretics, niacin, pentamidine, protease inhibitors and L-asparaginase), or illnesses such as myocardial infarction or stroke or forms of stress such as, e.g., after surgery and physical trauma. Further, hyperglycemia can be caused by infection and inflammation. All of the above diseases/forms of diabetes are in accordance with the invention subsumable under the term "diabetes", although from a clinical standpoint some of them may *strictu senso* not be considered to be subsumable under "diabetes", but rather a hyperglycemia.

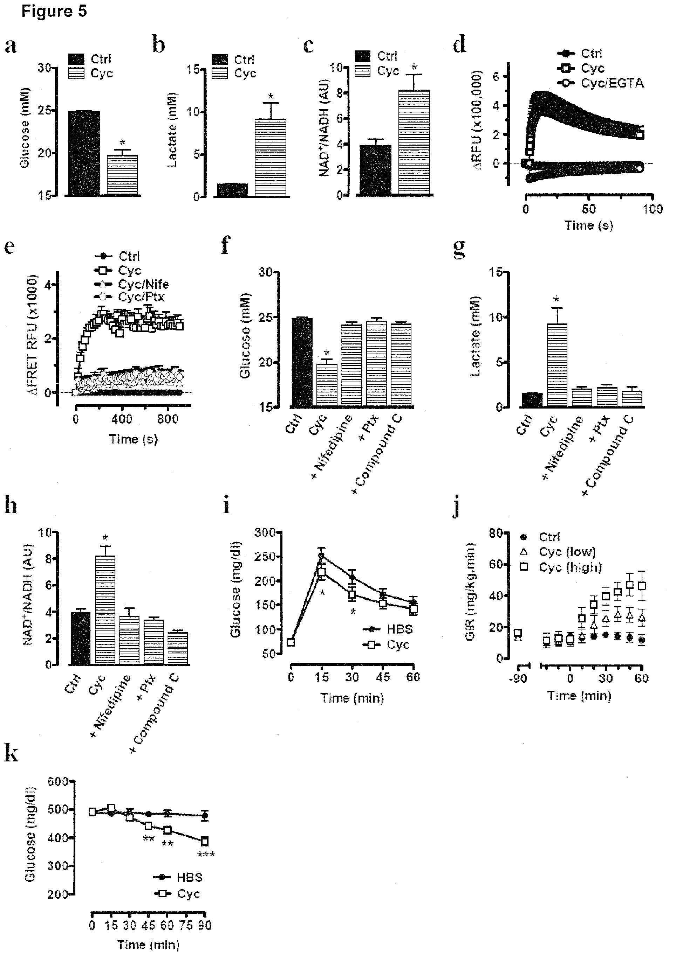

The present invention provides alternative means and methods to treat diabetes as is shown in the example section. It was surprisingly found that activators of the Smo receptor are capable to significantly and in a period of minutes increase glucose uptake, besides further factors involved in cellular energy metabolism. Briefly, while stimulating the Smo receptor with the Smoothened agonist (an activator) on 3T3-L1 adipocytes acidification of culture media and removal of glucose therefrom, as well as increased lactate accumulation elevated levels of the oxidized redox equivalents NAD⁺ ad NADP⁺ while NADH and NADPH remained unchanged could be observed. In fact, significant glucose removal and lactate generation was observed within minutes of SAG addition (cf., e.g., Example 2). Importantly, the kinetic, i.e. the rapid onset of metabolic rewiring is not consistent with canonical transcriptionally-driven hedgehog signalling. The same results could be obtained with the natural ligand Hedgehog signalling pathway sonic hedgehog (cf. Example 5). Thus, *in vitro* Hedgehog signalling involving the Smo receptor reprogrammed adipocyte metabolism towards aerobic glycolysis. Results could be replicated *in vivo* in mice. In detail, mice receiving 10 mg/kg cyclopamine as activator 15 minutes prior to testing displayed a moderate but significant improvement in glucose tolerance (Figure 5j). Neither basal nor glucose-stimulated insulin secretion appeared affected by the pre-treatment. Upon inactivation of the insulin secretory response (a physiological state mimicking type 1 diabetes) mice exhibited robust dose-dependent increases in glucose infusion rate within 10-20 minutes of cyclopamine infusion (Fig. 5j). These findings provide direct evidence that acute Smo-dependent metabolic rewiring occurs *in vivo.* In a type 1 diabetes mouse model, mice showed significant and robust glucose clearance within ∼30 minutes of cyclopamine injection (Fig. 5k), a remarkable stimulation of insulin independent glucose uptake *in vivo.* This further corroborates that the observed increase *inter alia* in glucose uptake upon stimulation of the Smo receptor and the non-canonical pathway occurs independent from insulin and insulin-mediated signalling.

Glucose intolerance also known as impaired glucose tolerance is a pre-diabetic state associated with insulin resistance that is characterized by periods of hyperglycemia. It is associated with increased cardiovascular pathology and increased mortality. Glucose intolerance is characterized by elevated 2 hour blood glucose levels 140 to 199 mg per dL (7.8 to 11.0 mmol) during a 75g oral glucose tolerance test. Insulin resistance is a condition characterized by a decreased responsiveness to insulin, similar to type 2 diabetes. Due to said decreased responsiveness to insulin, cellular glucose uptake is decreased and, as outlined above, any activator of the Smo receptor as defined herein above that is capable of increasing cellular glucose uptake will be effective in the treatment of said insulin resistance.

As exemplary activators of the Smo receptor in accordance with the invention the following activators are mentioned which can increase the glucose uptake of diabetic subject SAG (Smoothened Agonist), cyclopamine, or purmorphamine. SAG is a cell-permeable benzothiophene that activates Smo coupling to downstream signaling by interacting with the heptahelical domain (Kd -60nM). SAG competes with Cyclopamine, a naturally occurring plant steroidal alkaloid, for the same binding site on Smoothened (PNAS October 29, 2002 vol. 99 no. 22 14071-14076 J.K. Chen et al.). SAG and cyclopamine can be ordered from numerous commercial sources including ENZO life sciences and Merck.

Accordingly, in another embodiment, the invention relates to an activator of the Smo receptor for use in the treatment of diabetes, glucose intolerance, hyperglycemia, obesity, overweight, sepsis, and diseases associated therewith, wherein said activator is selected from one or more activators of the group consisting of SAG, cyclopamine, purmorphamine. Further compounds, also expected to be activators as defined herein are SANT-1, SANT-2, SANT-3, SANT-4, GDC-0449, NVP-LDE225, NVP-LEQ506, IPI609, IPI-926, BMS-833923, PF-04449913, TAK-441, Cur-61414, LY2940680, Itraconazol, Vitamin D.

In the course of the invention, it was furthermore surprisingly found that the canonical and non-canonical signalling pathway can be selectively regulated, i.e. uncoupled, at the level of the Smo receptor. The compound cyclopamine, previously known and used as classical inhibitor of hedgehog signalling binding to the Smo receptor, has been found to not only inhibit the canonical hedgehog signalling pathway, but to simultaneously activate the novel non-canonical hedgehog signalling pathway. Therefore, preferred activators are, but without limitation, activators that selectively activate the non-canonical signalling hedgehog pathway via the Smo receptor and not the canonical hedgehog signalling pathway, such as exemplarily, cyclopamine. Besides the generally accepted advantages of a selective pharmaceutically active agent from a pharmaceutical standpoint, further advantages of using corresponding selective activators is the observation that type 2 diabetes is clinically often linked to the occurrence of cancer and treatment of diabetes has been suggested to increase cancer rates due it being a growth factor (Giovannucci et al., Diabetes Care. 2010 Jul;33(7):1674-85. Diabetes and cancer: a consensus report.). Therefore, using cyclopamine may avoid the therapeutic use of insulin altogether or, as the case may be, if insulin is used as simultaneous therapy, it may decrease said cancer enhancing effect due to the antagonistic effect on the canonical hedgehog pathway. Also, it may be effective in patients afflicted with diabetes and cancer at the same time. It is understood that in accordance with the present invention, activators may activate both Hedgehog signalling pathways, i.e. the canonical and the non-canonical signalling pathway.

A further condition that can be treated on the basis of the increases in glucose uptake with activators in accordance with the present invention is sepsis. "Sepsis" is a condition well known in the art and relates to a whole-body inflammatory state and the presence of a known or suspected infection. As a hallmark of sepsis is the subject's incapability of glucose uptake into cells caused for example by development of insulin resistance. As will be understood from the above, the activators of Smo as defined herein are insulin independent means to increase glucose uptake. Therefore, if as treatment of sepsis glucose is administered to the subject afflicted with sepsis, glucose uptake is warranted. The term "sepsis" embraces all forms of sepsis, such as, e.g., the systemic inflammatory syndrome (SIRS), severe sepsis, or septic shock.

Two additional conditions that can be treated on the basis of the increases in glucose uptake achievable with activators in accordance with the present invention are obesity or the preobesity condition known as overweight. Overweight and obesity are conditions well known in the art and are characterized by increased levels of adipose tissue and typically diagnosed through measurement of body mass index (BMI = mass of the individual in kilograms divided by the squared height of the individual in meters). Overweight is defined as a BMI of 25-30 and Obesity by a BMI in excess of 30. Specifically, brown adipose tissue activity is inversely correlated with obesity. Markedly obese individuals typically have a near inactive brown adipose tissue mass. As brown adipose tissue burns and dissipates energy to form heat, activation of brown adipose tissue is a therapeutic strategy for those patients with residual activity in their brown adipose tissue. The invention demonstrates specific glucose uptake in brown adipose tissue in vivo in mice (cf. Example 5 and Fig .6) and therefore constitutes a method to specifically treat overweight and obese individuals displaying substantial residual activity in their brown adipose tissue.

There are also conditions where it is expected to be beneficial to slow down the rate of glucose uptake by those cells predominantly involved in glucose uptake such as, e.g., muscle cells. For example, hypoglycemia is a condition characterized by abnormally diminished blood glucose levels. Causes include hyperinsulinemia (excessive insulin secretion), genetic metabolism disorders, starvation, or acute chemical induction such as by medication, poison, alcohol. Medications can include for instance moderate over-dose of insulin or other glucose lowering agents. This leads predominantly an impairment of brain function, also termed neuroglycopenia. Thus, using the Smo receptor inhibitors as defined herein, one may inhibit glucose uptake, thereby minimizing decrease of the glucose in the blood before it reaches the brain. In other words, the glucose uptake is inhibited in the rest of the body and glucose is shunted to the brain.

Preferably, the modulator, i.e. the inhibitor or activator, in this embodiment of the present invention is comprised in a pharmaceutical composition, optionally further comprising a pharmaceutically acceptable carrier, excipient and/or diluent, The term "pharmaceutical composition", as used herein, relates to a composition for administration to a patient, preferably a human patient. The pharmaceutical composition of the invention comprises at least one, such as at least two, e.g. at least three, in further embodiments at least four such as at last five of the above mentioned inhibitors or activators. The invention also envisages mixtures of either inhibitors or activators of the Smo receptor falling into one or more of the different inhibitor or activator classes defined herein above. In cases where more than one inhibitor or activator is comprised in the pharmaceutical composition, it is understood that none of these inhibitors or activators has any essentially inhibitory effect on the other inhibitors or activators also comprised in the composition. This does not exclude the possibility that synergistic effects may be observed.

The composition may be in solid, liquid or gaseous form and may be, inter alia, in a form of (a) powder(s), (a) tablet(s), (a) solution(s) or (an) aerosol(s).

It is preferred that said pharmaceutical composition comprises a pharmaceutically acceptable carrier, excipient and/or diluent. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by delivery, e.g., to a site in the bloodstream such as a coronary artery or directly into the respective tissue, e.g., by injection. The compositions of the invention may also be administered directly to the target site, e.g., by biolistic delivery to an external or internal target site, like the muscles or brown adipose tissue. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 0.01 µg to 10 mg units per kilogram of body weight per minute. The continuous infusion regimen may be completed with a loading dose in the dose range of 1 ng and 10 mg/kg body weight.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. It is also envisaged that said pharmaceutical composition comprises further agents known in the art to be effective in the treatment of the above mentioned diseases. Since the pharmaceutical preparation of the present invention relies on the above mentioned inhibitors or activators, it is preferred that those mentioned further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

Also, the invention relates to a method of treating a disease by administering a modulator of the Smoothened (Smo) receptor to a subject in need thereof, wherein (a) the modulator is an activator of the Smoothened (Smo) receptor capable of increasing cellular glucose uptake and is for use in the treatment of diabetes, glucose intolerance, hyperglycemia, obesity, overweight, sepsis and diseases associated therewith; or (b) wherein the modulator is an inhibitor of the Smoothened (Smo) receptor capable of decreasing cellular glucose uptake and is for use in the treatment of hypoglycaemia. All definitions given herein, in particular above, also apply *mutatis mutandis* to this embodiment.

In a preferred embodiment, the diabetes is selected from type 1 diabetes, type 2 diabetes, type 3 diabetes, maturity onset diabetes of the young (MODY), neonatal diabetes, diabetes type 1.5, steroid-induced diabetes, pre-diabetes, gestational diabetes, type A insulin resistance, leprechaunism, Rabson-Mendenhall syndrome, lipoatrophic diabetes; diabetes associated with Down syndrome, Klinefelter syndrome, Turner syndrome, Wolfram syndrome, Friedreich ataxia, Huntington chorea, Laurence-Moon-Biedl syndrome, myotonic dystrophy, porphyria and Prader-Willi syndrome; diabetes caused by pancreatitis, trauma/pancreatectomy, neoplasia, cystic fibrosis, hemochromatosis, and/or fibrocalculous pancreatopathy; diabetes caused by Acromegaly, Cushing's syndrome, glucagonoma, pheochromocytoma, hyperthyroidism, somatostatinoma, and/or aldosteronoma; and diabetes that is induced by vacor, pentamidine, nicotinic acid, glucocorticoids, thyroid hormone, diazoxide, β-adrenergic agonists, thiazides, dilantin and/or γ-Interferon; diabetes cause by congenital rubella and/or cytomegalovirus infection.The above mentioned forms of diabetes are known in the art and have to some extent described herein above.

In another preferred embodiment, the diseases associated with diabetes are selected from the group consisting of obesity, hyperlipidemia, hypercholesterolemia, diabetic coma, diabetic ketoacidosis, hyperglycemia, diabetic cardiomyopathy, diabetic nephropathy, diabetic neuropathy, diabetic retinopathy, diabetic coronary artery disease, diabetic myonecrosis, peripheral vascular disease, and stroke.

The above-mentioned diseases associated with diabetes are known in the art. It is understood that treatment of diabetes has a treatment effect on these disease which are causally and/or mechanistically associated with diabetes as defined herein above.

In a further embodiment, the invention relates to a method of altering the glucose uptake, the lactate level, the NAD⁺/NADH ratio and/or the NADP⁺/NADPH ratio of a cell, said method comprising the step of (a) contacting said cell with a modulator of the Smoothened (Smo) receptor capable of altering glucose uptake, lactate level, NAD⁺/NADH ratio and/or NADP⁺/NADPH ratio, respectively.

The definitions given herein above also apply to this embodiment *mutatis mutandis.*

The cell in this embodiment can be any animal cell expressing the Smo receptor protein. Preferably, the cell endogenously expresses the Smo receptor protein, i.e. a functional receptor, but also recombinant expression is envisaged. It is understood by the person skilled in the art that depending on the goal to be achieved with the method described, different cells may be more suitable than others. Preferably, the cell is a mammalian cell, more preferred a human cell which is not a human embryonic stem cell. The term "mammalian cell" as used herein, is well known in the art and refers to any cell belonging to an animal that is grouped into the class of mammalia. The term "cell" as used herein can refer to a single and/or isolated cell or to a cell that is part of a multicellular entity such as a tissue, an organism or a cell culture. In other words the method can be performed *in vivo, ex vivo* or *in vitro.* The method preferably excludes methods of treatment of the human or animal body or diagnostic methods practised on the human or animal body. Depending on the particular goal to be achieved with the method of the invention, cells of different mammalian subclasses such as prototheria or theria may be used. For example, within the subclass of theria, preferably cells of animals of the infraclass eutheria, more preferably of the order primates, artiodactyla, perissodactyla, rodentia and lagomorpha are used in the method of the invention. Furthermore, within a species one may choose a cell to be used in the method of the invention based on the tissue type and/or capacity to differentiate equally depending on the goal to be achieved by altering the genome. Three basic categories of cells make up the mammalian body: germ cells, somatic cells and stem cells. A germ cell is a cell that gives rise to gametes and thus is continuous through the generations. Stem cells can divide and differentiate into diverse specialized cell types as well as self renew to produce more stem cells. In mammals there are two main types of stem cells: embryonic stem cells and adult stem cells. Somatic cells include all cells that are not a gametes, gametocytes or undifferentiated stem cells. The cells of a mammal can also be grouped by their ability to differentiate. A totipotent (also known as omnipotent) cell is a cell that is able to differentiate into all cell types of an adult organism including placental tissue such as a zygote (fertilized oocyte) and subsequent blastomeres, whereas pluripotent cells, such as embryonic stem cells, cannot contribute to extraembryonic tissue such as the placenta, but have the potential to differentiate into any of the three germ layers endoderm, mesoderm and ectoderm. Multipotent progenitor cells have the potential to give rise to cells from multiple, but limited number of cell lineages. Further, there are oligopotent cells that can develop into only a few cell types and unipotent cells (also sometimes termed a precursor cell) that can develop into only one cell type. There are four basic types of tissues: muscle tissue, nervous tissue, connective tissue and epithelial tissue that a cell to be used in the method of the invention can be derived from, such as for example hematopoietic stem cells or neuronal stem cells. To the extent human cells are envisaged for use in the method of the invention, it is preferred that such human cell is not obtained from a human embryo, in particular not via methods entailing destruction of a human embryo. On the other hand, human embryonic stem cells are at the skilled person's disposal such as taken from existent embryonic stem cell lines commercially available. Accordingly, the present invention may be worked with human embryonic stem cells without any need to use or destroy a human embryo. Alternatively, or instead of human embryonic stem cells, pluripotent cells that resemble embryonic stem cells such induced pluripotent stem (iPS) cells may be used, the generation of which is state of the art (Hargus G et al., Proc Natl Acad Sci U S A 107:15921-15926; Jaenisch R. and Young R., 2008, Cell 132:567-582; Saha K, and Jaenisch R., 2009, Cell Stem Cell 5:584-595.

Exemplary cell types that may be used in accordance with the invention, one may use muscle cells, fibroblasts, white adipocytes or brown adipocytes.

Cells to be used may originate from established cell lines but may also include cell of a primary cell line established from a tissue sample. Preferably, the cells originate from the same cell line or are established from the same tissue. Methods to obtain samples from various tissues and methods to establish primary cell lines are well-known in the art (Jones GE, Wise CJ., "Establishment, maintenance, and cloning of human dermal fibroblasts." Methods Mol Biol. 1997;75:13-21). Suitable cell lines may also be purchased from a number of suppliers such as, for example, the American tissue culture collection (ATCC), the German Collection of Microorganisms and Cell Cultures (DSMZ) or PromoCell GmbH, Sickingenstr. 63/65, D-69126 Heidelberg.

As outlined above, certain cells may be more suitable than others to achieve the modification of the glucose uptake, the lactate level, the NAD⁺/NADH ratio and/or the NADP⁺/NADPH ratio of a cell. For example, the phenotype and physiological state of a specific cell may be more suitable to achieve a pronounced modification of the glucose uptake than to alter lactate levels. For example, and with reference to an activator as modulator for increasing glucose uptake particularly suitable cells include, e.g., muscle cells, white or brown adipocytes (such as C2C12 or 3T3-L1 cells); for increasing lactate levels particularly suitable cells include, e.g., fibroblasts, adipocytes (such as mouse embryonic fibroblasts and 3T3-L1 cells); for increasing the NAD⁺-level (thereby altering the NAD⁺/NADH ratio) particularly suitable cells include, e.g., muscle cells, fibroblasts, adipocytes (such as mouse embryonic fibroblasts, C2C12 and 3T3-L1 cells); for increasing the NADP⁺-level (thereby altering the NADP⁺/NADPH ratio) particularly suitable cells include, e.g., muscle cells, fibroblasts, adipocytes (such as mouse embryonic fibroblasts, C2C12 and 3T3-L1 cells).

It is further understood that due to said differences in phenotype and physiological state one may not be in the position to experimentally determine a modification of all biological functions or activities of the Smo receptor defined herein, e.g,. glucose uptake, the lactate level, the NAD⁺/NADH ratio and/or the NADP⁺/NADPH ratio in a given cell. Preferably, the altered biological functions or activities of the Smo receptor as defined herein include at least increased glucose uptake and, optionally, any combination of changes or lack thereof in lactate, NAD⁺/NADH ratio and/or the NADP⁺/NADPH ratio. For example, altered biological functions or activities of the Smo receptor as defined herein refer to altered glucose uptake and lactate level; altered glucose uptake and NAD⁺/NADH ratio; altered glucose uptake and NADP⁺/NADPH ratio; altered glucose uptake, lactate level and NAD⁺/NADH ratio; altered glucose uptake, lactate level and NADP⁺/NADPH ratio; or altered glucose uptake, NAD⁺/NADH ratio and NADP⁺/NADPH ratio.

It is also understood by the person skilled in the art that not each modulator will exhibit the same quantitative and qualitative response. Thus, also in this regard the skilled person has a choice depending on the specific goal to be achieved with the present method.

In a preferred embodiment of the method of the invention, the method further comprises a step (b) of contacting the cell of step (a) with glucose or a glucose analogue labelled with a detectable moiety prior to, simultaneously with or after having contacted said cell of step (a) with said modulator of the Smoothened (Smo) receptor capable of altering glucose uptake.

The person in the art knows the atomic composition of glucose as well as methods to produce the monosaccharide glucose. Further, he knows that the term "glucose analogue" relates to molecules based on the structure of glucose where one or several chemical moieties have been added or modified. Preferably, one or more of the OH-groups are substituted. Glucose analogues include, e.g., 2-fluoro-deoxy-D-glucose, 2-deoxy-D-glucose, α-methyl-d-glucoside and β-methyl-d-glucoside, 2-deoxy-2-fluoro-D-mannose, and 2,2-difluoro-deoxy-D-glucose.

The term "detectable moiety" as used herein relates to a molecular entity whose presence (and absence) can be established by any suitable means. The skilled person is aware of various detectable moieties that are known in the art for labelling molecules such as glucose or glucose analogues. The detectable label may be any label that is detectable using known detection methods, including imaging methods. For example, the detectable moiety may include a luminophore (emitting fluorescent or phosphorescent light) and may also be a detectable moiety suitable for and/or known to be used in positron emission tomography (PET), a detectable moiety suitable for and/or known to be used in single-photon emission computed tomography (SPECT), a detectable moiety suitable for and/or known to be used in magnetic resonance imaging (MRI), a quantum dot, a coloured label, a radiolabel, an enzyme which converts a substrate that can be detected or a detectable moiety that may be detected by an antibody or antibody fragment. Such detectable moieties are known and are readily available. For example, fluorescent moieties include BODIPY, FITC, Rhodamine, TRITC, Texas Red, cyanine dyes (e.g. Cy3 or Cy5) or Alexa fluors. Radiolabels include moieties or groups having at least one radioactive isotope, and include moieties or groups having a positron emitting radioactive isotope or a gamma emitting radioactive isotope. Detectable moieties further include additions such as methyl groups or isotopic atoms (radionuclides) themselves (strontium-82, rubidium-82, carbon-11 (¹¹C), oxygen-15 (¹⁵O), fluorine-18 (¹⁸F)), which can also constitute glucose analogues when replacing an atomic constitute of glucose, e,g., replacement of the lost oxygen atom of deoxy-glucose. Examples of radionuclides are tritium, carbon-14, sodium-22, sulfur-35, phosphorus-33, phosphorus-32, and iodine-125, In particular, radionuclides (atoms with an unstable nucleus) such as useful for PET scanning may include an unstable positron-emitting isotope. Such isotopes may be synthesized in a cyclotron by bombarding nitrogen, carbon, oxygen, or fluorine with protons. Examples of the isotopes used for PET labels include ¹⁵O (half-life: 2 min), ¹⁸F (half-life: 110 min), and ¹¹C (half-life: 20 min). Preferably, a corresponding detectably labelled glucose analogue for use in PET is fluorodeoxyglucose (¹⁸F) (abbreviated as FDG or F-FDG) which chemically is 2-deoxy-2-(¹⁸F)fluoro-D-glucose with the positron-emitting radioactive isotope fluorine-18 substituted for the normal hydroxyl group at the 2' position in the glucose molecule. Positron or photon emitting atoms such as 18F; 11C, 1251, 1231, 16N, 15O, 3H, 133Xe, 111In, 68Ga and other isotopes of metals such as technetium, or copper may be used in PET labels or SPECT labels. MRI detectable moieties include T1 (Gd) and T2 (Fe3O4) contrast agents.

Preferably, detection of the detectable moiety is possible in a concentration-dependent manner. The glucose or glucose analogue may be labelled anywhere and in any fashion as long detection of the detectable moiety is warranted, e.g., 3-*O*-¹¹C-methyl-D-glucose, 3-¹⁸F-fluoro-D-glucose, 3-*O*-¹¹C-methyl-D-glucose, 2-deoxy-2-¹²³I-iodo-D-mannose, 2-deoxy-2-¹⁸F-fluoro-D-glucose.

Depending on the goal to be achieved with the method of the invention, one may opt to execute step (b) prior to step (a), at the same time as step (a) or after step (a). In other words, the incrementing designation of the steps as (a) and (b) does not necessarily mean that step (a) is executed before step (b).

In a further embodiment, the invention relates to a method of identifying a modulator of the Smoothened (Smo) receptor capable of altering the glucose uptake, the lactate level, the NAD⁺/NADH ratio and/or the NADP⁺/NADPH ratio of a cell, comprising the steps of: (a) determining the level of glucose uptake, the level of lactate, the NAD⁺/NADH ratio and/or the NADP⁺/NADPH ratio in a cell containing Smo receptors; (b) contacting said cell or a cell of the same cell population with a test compound; (c) determining the level of glucose uptake, the level of lactate, the NAD⁺/NADH ratio and/or the NADP⁺/NADPH ratio in said cell after contacting with the test compound; (d) comparing the level of glucose uptake, the level of lactate, the NAD⁺/NADH ratio and/or the NADP⁺/NADPH ratio determined in step (c) with the glucose uptake level, the lactate level, the NAD⁺/NADH ratio and/or the NADP⁺/NADPH ratio determined in step (a) or, alternatively or additionally, with established standard values; and (e) determining prior to, simultaneously with or after any one of the preceding steps (a) to (d) whether said test agent modulates the Smo receptor, wherein a change of the glucose uptake level, a change of the lactate level, a change of the NAD⁺/NADH ratio and/or a change of the NADP⁺/NADPH ratio in step (c) as compared to step (a) and modulation of the Smo receptor by the test compound indicates that said test compound is a modulator of the Smo receptor capable of altering the glucose uptake, the lactate level, the NAD⁺/NADH ratio and/or the NADP⁺/NADPH ratio of a cell.

This embodiment relates to a cellular screen, wherein modulators may be identified which exert their altering activity by physically interacting with the Smo receptor to modulate the latter, or alternatively (or additionally) by functionally interacting with the Smo receptor, i.e., by interfering with the pathway(s) present in the cells employed in the cellular assay, preferably upstream of the Smo receptor, to modulate said Smo receptor. Furthermore, such compounds may, as described above, alter the affinity or rate of binding of a known ligand, such as sonic hedgehog, to the receptor or compete with a ligand for binding to the receptor or displace a ligand bound to the receptor. As a result, one or more of the specific biological functions or activities of the Smo receptor (glucose uptake, lactate level, NAD⁺/NADH ratio and/or NADP⁺/NADPH ratio) are modified either directly or indirectly, which can be measured as an altered level of one ore more of the specific biological functions or activities mentioned.

The term "said cell or a cell of the same cell population" as used herein refers either to the cell used in step (a) or to a cell being of the same origin as the cell of step (a) and that is identical in its characteristics to the cell of (a). Furthermore, this term also encompasses cell populations, such as for example homogenous cell populations consisting of cells having identical or essentially identical characteristics, and, thus, is not restricted to single cell analyses. The same limitations and definitions with regard to the term "cell" given herein above apply also to this embodiment *mutatis mutandis.*

"Established standard values" are values that have previously been generated in the respective cells used in the assay and are at the disposal of the person implementing the method of the invention. A corresponding setup may prove beneficial in particular in view of high throughput screenings (HTS).

As described hereinabove, the Smo receptor plays a heretofore unknown key role in cellular energy metabolism. Therefore, the use of the Smo receptor as a target for the identification of modulators of the latter as defined herein, which may be suitable as a lead compound and/or for the treatment of the above specified diseases, is also encompassed by the present invention. It is envisaged that an alteration in the glucose uptake level, a change of the lactate level, a change of the NAD⁺/NADH ratio and/or a change of the NADP⁺/NADPH ratio conferred by a test compound acting as modulator as described above will contribute to the treatment of the diseases referred to herein above and may ameliorate diseases associated therewith, as described above. Accordingly, measurement of the level of glucose uptake, the level of lactate, the NAD⁺/NADH ratio and/or the NADP⁺/NADPH ratio in a cell containing Smo receptors may be used as a readout of the above-described assay.

For example, the above-mentioned cell containing Smo receptors may exhibit a detectable or no level of, e.g., glucose uptake before contacting with the test compound and the level of glucose uptake may be increased after contacting the cell with the test compound, indicating that an activator suitable for the treatment of the diseases mentioned above or as a lead compound for the development of a compound for the treatment of the latter disease has been identified. Preferably, the glucose uptake level, the lactate level, the NAD⁺/NADH ratio and/or the NADP⁺/NADPH ratio after contacting the cell with the test compound is altered by, for example, at least 10, at least 20, at least 30, at least 40 or at least 50% as compared to the same readout parameters before contacting the cell with the test compound. More preferred, the glucose uptake level, the lactate level, the NAD⁺/NADH ratio and/or the NADP⁺/NADPH ratio after contacting the cell with the test compound is altered by, for example, at least 60, at least 70, at least 80, at least 90 or at least 95% as compared to the same readout parameters before contacting the cell with the test compound. Most preferred, and in the case of the test agent displaying characteristics of an inhibitor, the glucose uptake level, the lactate level, the NAD⁺/NADH ratio and/or the NADP⁺/NADPH ratio after contacting the cell with the test compound is reduced by 100% as compared to the same readout parameters before contacting the cell with said test compound. Most preferred, and in the case of the test agent displaying characteristics of an activator, the glucose uptake level, the lactate level, the NAD⁺/NADH ratio and/or the NADP⁺/NADPH ratio after contacting the cell with the test compound is increased by 100%, preferably more than 100% such as 150%, 200%, 500% or 1000% as compared to the same readout parameters before contacting the cell with said test compound. The term "the glucose uptake level, the lactate level, the NAD⁺/NADH ratio and/or the NADP⁺/NADPH ratio is changed by (at least)...%" refers to a relative alteration compared to the glucose uptake level, the lactate level, the NAD⁺/NADH ratio and/or the NADP⁺/NADPH ratio before contacting the cell with the test compound. For example, a reduction of at least 40% means that after contacting the cell with the test compound the remaining glucose uptake level, lactate level, NAD⁺/NADH ratio and/or NADP⁺/NADPH ratio is only 60% or less as compared to the glucose uptake level, the lactate level, the NAD⁺/NADH ratio and/or the NADP⁺/NADPH ratio before contacting the cell with the test compound.

In order to ensure that the observed change of the glucose uptake level, of the lactate level, of the NAD⁺/NADH ratio and/or of the NADP⁺/NADPH ratio results from the altering action of a Smo receptor modulating test compound, it has to be determined whether the test compound actually modulates the Smo receptor. As described herein above in relation to inhibitors and activators there exist various ways an inhibitor or activator can modulate the Smo receptor such as affecting Smo receptor protein translation as well as Smo receptor gene transcription or direct and/or indirect interaction with the Smo receptor protein. Said step (e) of whether the test compound modulates the Smo receptor can be performed at any stage of the method according to the invention. In some instances it may be worthwhile to carry out step (e) as initial step to narrow down the test compounds only to those that modulate Smo. Alternatively, step (e) may be performed as the final step of the method of the invention, so as to further screen the other test compounds that were capable to alter any one of the glucose uptake level, a change of the lactate level, a change of the NAD⁺/NADH ratio and/or a change of the NADP⁺/NADPH ratio can further be screened for their molecular mechanism of action that does not involve a modulation of the Smo receptor.

As outlined herein above, the alteration in the biological function or activity by a Smo modulator in accordance with the invention can, preferably, be observed within minutes after contact with said modulator. Therefore, it is preferred that step (c) of the method of the invention is carried out within the same time frames as defined herein above when the alteration can first be observed. Also preferred is a continuous determination of the level of glucose uptake, the level of lactate, the NAD⁺/NADH ratio and/or the NADP⁺/NADPH ratio in said cell after contacting with the test compound. A continuous determination may be in the form of a real time, i.e. uninterrupted, determination or be in the form of several consecutive measurements at the same or varying time intervals.

In a preferred embodiment, the method is carried out *in vitro. In vitro* methods offer the possibility of establishing high-throughput assays, as described above.

In a further preferred embodiment of the method of the invention, step (e) comprises: (a) assessing whether said test compound binds to the Smo receptor or a cellular activator of the Smo receptor, wherein binding of said test compound to said Smo receptor or said cellular activator of the Smo receptor indicates that said test compound modulates the Smo receptor; and/or (b) determining the level of Smo receptor protein or Smo receptor transcript in a cell according to step (a) of the method of identifying described herein above; (c) determining the level of Smo receptor protein or Smo receptor transcript in said cell according to step (b) of the method of identifying described herein above after contact with said test compound; and (d) comparing the level of Smo receptor protein or Smo receptor transcript determined in step (c) with the Smo receptor protein or Smo receptor transcript level determined in step (a), wherein a change of Smo receptor protein or Smo receptor transcript level in step (c) as compared to step (a) indicates that the test compound modulates the Smo receptor.

As outlined herein above, it is necessary to determine whether the test compound actually modulates the Smo receptor to ascertain that the altered biological functions or activities detected in the course of executing the method of the invention are causally related to the non-canonical Hedgehog signalling pathway.

Hence, step (e) may involve a step (a) where it is determined whether the test compound binds to the Smo receptor or an activator of the Smo receptor. The term "cellular activator of the Smo receptor" relates to an entity that is endogenously present in the cell containing the Smo receptor and is naturally involved in the recruitment of the Smo receptor, such as e.g., the Patched (Ptch) receptor, beta-arrestin, or G-protein coupled receptor kinase 2 (GRK2). As described herein above various methods exist to assess the binding of two molecular entities to each other.

As an alternative, or additionally, step (e) may involve steps (b) to (d) where it is determined whether the test compound modulates the Smo receptor by altering the levels of the Smo receptor protein or the Smo receptor transcript. For example, the above-mentioned cell may exhibit a detectable level of Smo receptor protein or Smo receptor transcript before contacting with the test compound and the level of Smo receptor protein or Smo receptor transcript may be lower or undetectable after contacting the cell with the test compound, indicating that the test compound modulates the Smo receptor by decreasing it quantitatively and that it may affect the biological function or activity of the Smo receptor in the sense of an inhibitor as defined herein above. On the other hand, an increase in the level of Smo receptor protein or Smo receptor transcript after contacting the cell with the test compound indicates that the test compound modulates the Smo receptor by increasing it quantitatively and that it may affect the biological function or activity of the Smo receptor in the sense of an activator as defined herein above. Measurements of protein levels as well as of transcript level can be accomplished in several ways, as described above.

Preferably, for all embodiments, the test compound or the modulator of the Smo receptor are active site binders on the Smo receptor or do not bind directly to the target, but still interfere with its function or activity, for example by binding to and/or activating the function or increasing the expression of members of a pathway which comprises the target, i.e. the Smo receptor. Preferably, modulators indirectly modulating the Smo receptor are interacting with cellular molecules upstream of the Smo receptor.

In another preferred embodiment of the modulator or the method of the invention, the modulator or the test compound is an antibody or a fragment or derivative thereof, an aptamer, an siRNA, an shRNA, a miRNA, a ribozyme, an antisense nucleic acid molecule, a small molecule or modified versions of these modulators or test compounds .

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments as well as Fd, F(ab')₂, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies.

Various techniques for the production of antibodies are well known in the art and described, e.g. in Harlow and Lane (1988) and (1999), loc. cit. Thus, the antibodies can be produced by peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique (originally described by Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to a target (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys- loop in the wild protein, the two cysteins lateral chains being able to form a disulfide bridge. Peptide aptamer selection can be made using different systems, but the most widely used is currently the yeast two-hybrid system. Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are usually cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The term "peptide" as used herein describes a group of molecules consisting of up to 30 amino acids, whereas the term "polypeptide" as used herein describes a group of molecules consisting of more than 30 amino acids. The group of peptides and polypeptides are referred to together with the term "(poly)peptide". Also encompassed by the term "(poly)peptide" are proteins as well as fragments of proteins of more than 30 amino acids. The term "fragment of protein" in accordance with the present invention refers to a portion of a protein comprising at least the amino acid residues necessary to maintain the biological activity of the protein. Preferably, the amino acid chains are linear. (Poly)peptides may further form multimers consisting of at least two identical or different molecules. The corresponding higher order structures of such multimers are correspondingly termed homo- or heterodimers, homo- or heterotrimers etc. Furthermore, peptidomimetics of such (poly)peptides where amino acid(s) and/or peptide bond(s) have been replaced by functional analogues are also encompassed by the invention. Such functional analogues include all known amino acids other than the 20 gene-encoded amino acids, such as selenocysteine. The term "(poly)peptide" also refers to naturally modified (poly)peptides where the modification is effected e.g. by glycosylation, acetylation, phosphorylation and similar modifications which are well known in the art.

It is also well known that (poly)peptides are not always entirely linear. For instance, (poly)peptides may be branched as a result of ubiquitination, and they may be circular, with or without branching, generally as a result of post-translation events, including natural processing event and events brought about by human manipulation which do not occur naturally. Circular, branched and branched circular (poly)peptides may be synthesized by non-translational natural processes and by synthetic methods. The modifications can be a function of how the (poly)peptide is made. For recombinant (poly)peptides, for example, the modifications will be determined by the host cells posttranslational modification capacity and the modification signals in the amino acid sequence. Accordingly, when glycosylation is desired, a (poly)peptide should be expressed in a glycosylating host, generally an eukaryotic cell, for example Cos7, HELA or others. The same type of modification may be present in the same or varying degree at several sites in a given (poly)peptide. Also, a given (poly)peptide may contain more than one type of modification.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics). The activity and specificity of siRNAs can be altered by various modifications such as, e.g., by inclusion of a blocking group at the 3' and 5' ends, wherein the term "blocking group refers to substituents of that can be attached to oligonucleotides or nucleomonomers, either as protecting groups or coupling groups for synthesis (cf. WO 98/13526, EP 2221377 B1), by inclusion of agents that enhance the affinity to the target sequence such as intercalating agents (e.g., acridine, chlorambucil, phenazinium, benzophenanthirdine), attaching a conjugating or complexing agent or encapsulating it to facilitate cellular uptake, or attaching targeting moieties for targeted delivery.

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed, e.g., as an inhibitor of the Smo receptor.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and *in vitro*-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity *in vitro* and in some cases also *in vivo.* The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

A "small molecule" as used herein may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in *in vivo* assays such as *in vivo* high-throughput screening (HTS) assays. Preferred small molecules are described herein and include SAG and cyclopamine as well as the further small molecules referred to herein below.

The term "modified versions of these modulators or test compounds" in accordance with the present invention refers to versions of the modulators or test compounds that are modified to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (I) derivatisation of hydroxyl groups to ketales, acetales, or (m) N-acetylation to amides, phenylcarbamates, or (n) synthesis of Mannich bases, imines, or (o) transformation of ketones or aldehydes to Schiff's bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

In another embodiment, the invention relates to a method of increasing uptake of glucose or a glucose analogue labelled with a detectable moiety into a cell, said method comprising the steps of: (a) contacting said cell with an activator of the Smoothened (Smo) receptor capable of increasing glucose uptake, and (b) contacting the cell of step (a) with glucose or a glucose analogue labelled with a detectable moiety prior to, simultaneously with or after having contacted said cell of step (a) with said activator of the Smoothened (Smo) receptor capable of increasing glucose uptake.

The definitions given herein above also apply *mutatis mutandis* to this embodiment. There are various scenarios in which detectably labelled glucose or analogues thereof are useful when taken up at an enhanced rate into a cell. In particular in diagnostic imaging methods such as, e.g., PET it may prove beneficial to achieve a detectable signal in cells that usually do not or not as much take up the labelled glucose or analogue thereof used as imaging agent. Also, it may enhance the signal quality and limit the amounts needed to be administered to a subject. The latter aspect may reduce costs and may further decrease the number and severity of side effects occurring in patients that prove to exhibit an allergic reaction to the imaging reagent. Thus, patients that may previously be unsuitable for receiving the imaging agent may become eligible due to the decrease in the amount of imaging reagent necessary.

In a preferred embodiment of the methods of the invention, the detectable moiety is selected from the group consisting of a radionuclide, a luminophore or an enzyme as mentioned herein above.

In a more preferred embodiment of the method of the invention, the glucose or glucose analogue labelled with a radionuclide is suitable for use as an imaging agent in positron emission tomography (PET). As outlined herein above, the skilled person knows labelled glucose molecules or analogues thereof suitable for use as imaging reagent. "Suitable" are any labelled glucose molecules or analogues that can be safely administered to a subject and are capable of being detected and having their signal translated into an image.

In a preferred embodiment of the modulator or the methods of the invention, the modulator or activator binds to the Smo receptor or an activator of the Smo receptor. In line with the foregoing, it is preferred that the modulator, in particular if an agonist, binds directly to the Smo receptor or an activator thereof as defined herein above. Preferably, the binding occurs with the heptahelical bundle of the Smo receptor and may further not require the cytoplasmic tail or CDR for binding. Corresponding binders are, e.g., SAG and cyclopamine (Chen et al., PNAS October 29, 2002 vol. 99 no. 22 14071-14076).

In a more preferred embodiment of the modulator or the methods of the invention, the activator or the modulator, in case it is an activator of the Smo receptor, is selected from the group consisting of SAG, cyclopamine, purmorphamine, SANT-1, SANT-2, SANT-3, SANT-4, GDC-0449, NVP-LDE225, NVP-LEQ506, IPI609, IPI-926, BMS-833923, PF-04449913, TAK-441, Cur-61414, LY2940680, Itraconazol, or Vitamin D. Also encompassed are analogues and derivatives of the above recited compounds that display the function of an activator or inhibitor of the Smo receptor as defined herein.

The compounds mentioned above are well-known in the art. A brief summary is given below for each compound.

The activator "SAG" (abbreviation of Smoothened Agonist) has the CAS Number: 364590-63-6; the molecular weight is: 599.0; the molecular formula is: C₂₈H₂₈CIN₃OS•2HCl•2H₂O. Cyclopamine (also 11-deoxojervine) has the CAS Number: 4449-51-8; the molecular weight is: 411.62; the molecular formula is: C₂₇H₄₁NO₂.

*SANT-1* has the CAS Number: 304909-07-7; the molecular weight is 373.5; the molecular formula: C₂₃H₂₇N₅.

*SANT-2* has the CAS Number: 329196-48-7; the molecular weight is 480.0; the molecular formula is C₂₆H₂₆ClN₃O₄.

*SANT-3* and *SANT-4* have the following formulae

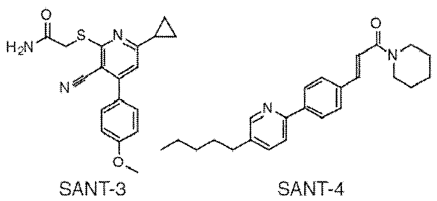

*Puromorphamine* has the CAS Number: 483367-10-8; the molecular weight is 520.6; the molecular formula is C₃₁H₃₂N₆O₂.

*HhAntag (=GDC-0449)*

*GDC-0449 (Vismodegib, HhAntag691)* has the CAS Number: 879085-55-9; the molecular weight is 421.3; the molecular formula is C₁₉H₁₄Cl₂N₂O₃S.

*NVP-LDE225* has the CAS Number 956697-53-3; the molecular weight is 485.5; the molecular formula is C₂₆H₂₆F₃N₃O₃.

*NVP-LEQ506* is described as part of National Institute of Health (NIH) Clinical Trial Identifier No. NCT01106508.

*IPI-926 (Saridegib)* has the CAS Number: 1037210-93-7; the molecular weight is 504.77; the molecular formula is C₂₉H₄₈N₂O₃S.

*BMS-833923 (XL139)* is described as part of National Institute of Health Clinical Trial Identifier No. NCT006701891

*PF-04449913* is described as part of National Institute of Health Clinical Trial Identifier No. NCT00953758.

*TAK-441* is described as part of National Institute of Health Clinical Trial Identifier No.NCT01204073.

*Cur-61414* has the CAS Number: 334998-36-6; the molecular weight is 550.69; the molecular formula is C₃₁H₄₂N₄O₅.

*Itraconazol* has the CAS Number: 84625-61-6; the molecular weight is 705.64; the molecular formula is C₃₅H₃₈Cl₂N₈O₄.

*Vitamin D (Cholecalciferol)* has the CAS Number: 67-97-0; the molecular weight is 384,64; the molecular formula is C₂₇H₄₄O.

*MS-0022* has the molecular weight is 198.26; the molecular formula is C₉H₁₁FN₂S. *Cyclopamine-KAAD* has the CAS Number: 306387-90-6; the molecular weight is 697.99; the molecular formula is C₄₄H₆₃N₃O₄.

*IPI-609* is described in US Patent Application Publication No. US 2011/0135739 A1 (Carter et al.) and has the formula

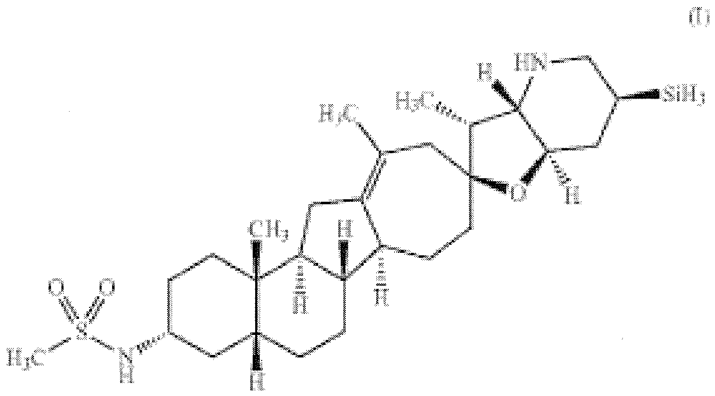

In another preferred embodiment of the modulator or the methods of the invention, the activator or modulator, in case it is an activator of the Smo receptor, is further capable of activating G-proteins via the Smo-receptor, phosphorylating Amp-activated protein kinase (Ampk) and/or opening Ca²⁺-channels.

While alterations in the glucose uptake level, the lactate level, the NAD⁺/NADH ratio and/or the NADP⁺/NADPH ratio are described herein to constitute biological functions and activities of the Smo receptor, there are further biological functions and activities of the Smo receptor that are altered upon modulation of the Smo receptor with an activator or inhibitor. It is of note that some of said further biological functions and activities mediate alterations of the glucose uptake level, of the lactate level, of the NAD⁺/NADH ratio and/or of the NADP⁺/NADPH ratio, as they interact with each other directly or indirectly as part of the non-canonical Hedgehog signalling pathway that has been identified and forms the basis of the invention.

For example, activators of the Smo receptor as defined herein above are capable of activating G-proteins. G-proteins are one of the largest families (∼950 members) of intracellular signaling proteins. Their catalysis of GTP hydrolysis links cell surface receptor activation to a plethora of intracellular processes whose specificity is defined in part by the array of classes and sub-types of G-proteins including for example for G_{α} subunits: Gₛα (stimulatory), Gᵢα (inhibitory), Gₒα (other), and many more. They couple ligand-receptor binding to intracellular function of virtually every known hormone in the body. Further, they are capable of phosphorylating the AMP-kinase. AMPK is a master regulator of energy metabolism whose activation can lead (depending on cellular context) to glycogen synthesis, glycolysis, glucose transporter expression, cell growth, lipolysis, fatty acid oxidation and multiple other end-points of carbohydrate, lipid and protein metabolism. The majority of these endpoints counteract the processes of metabolic diseases such as aging, obesity, cardiovascular disease, and insulin resistance. An exemplary method for detecting phosphorylation of AMPK is described herein below in the example section. Moreover, activators of the Smo receptor as defined herein are also capable of triggering the opening of Calcium channels to allow recruitment of the second messenger Ca²⁺. As an exemplary method to determine Ca²⁺-channel opening the calcium flux can be measured as described in the example section.

The figures show:

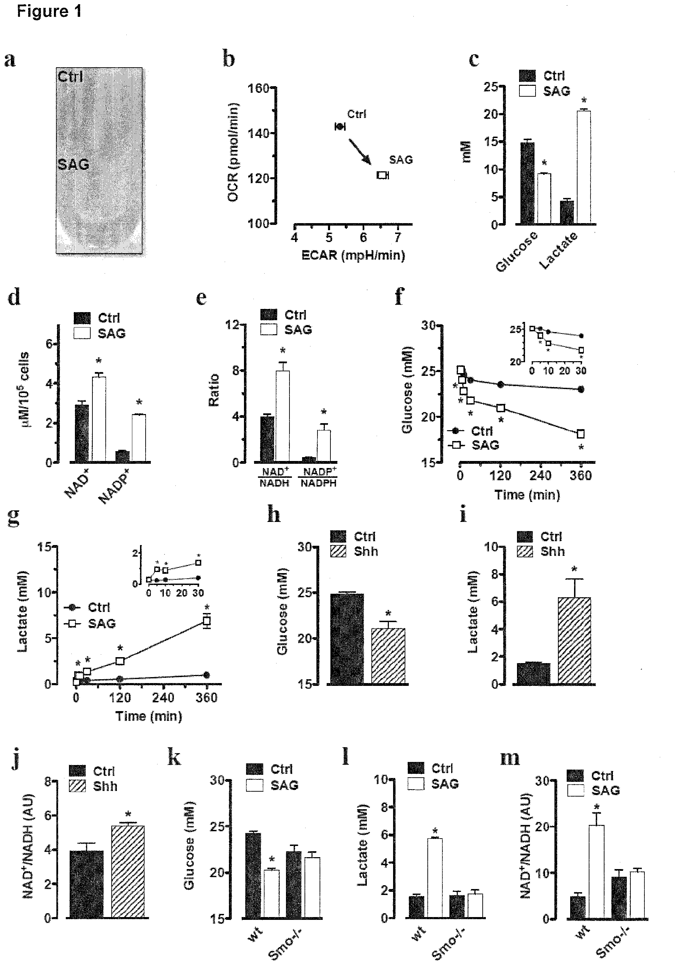

**Figure 1****: Hedgehog reprograms adipocyte metabolism**
   **a** Differentiated 3T3-L1 adipocytes treated with SAG (200 nM) acidify growth medium; b display reduced O₂-consumption rate (OCR) and increased extracellular acidification rate (ECAR); **c** utilize glucose and produce more lactate; **d** and show increased NAD⁺, NADP⁺; and **e** their ratios within 24 hours of treatment. **f** SAG-treated 3T3-L1 adipocytes increase glucose consumption and **g** lactate production within minutes of SAG addition. **h** Differentiated 3T3-L1 adipocytes treated with Sonic Hedgehog (Shh) show increased glucose consumption, **i** lactate production and **j** elevated NAD⁺/NADH. **k** Unlike wild-type (wt) MEFs, Smo^{-/-} MEFs are not responsive to SAG in terms of glucose consumption; **I** lactate production; **m** or change in NAD⁺/NADH ratio. Results are mean ± s.e.m. of at least three independent experiments performed in quadruplicate. * = p < 0.05 (two-tailed Student's t-test).

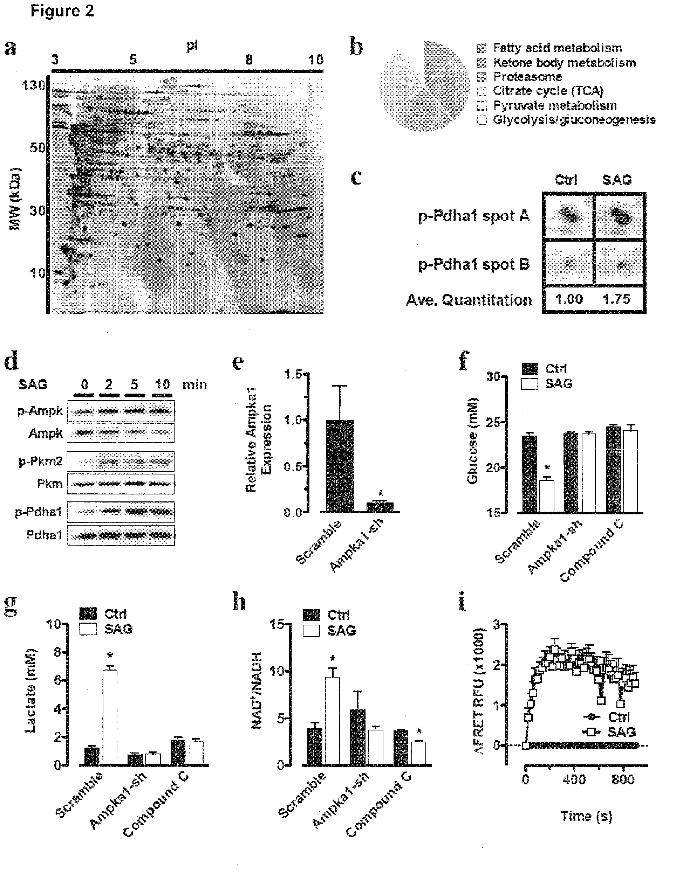

**Figure 2****: Phospho-proteomics and Ampk-dependent metabolic reprogramming**
   **a** Fluorescence-stained phospho-2D-Gel from 3T3-L1 adipocytes treated with SAG for 10 minutes. Highlighted spots are those significantly altered in 2 of 3 independent experiments. **b** KEGG pathway analysis of differentially regulated phospho-proteins. **c** Two significantly phosphorylated spots corresponding to Pdha1. **d** Western blot time course of SAG-induced protein phosphorylation in 3T3-L1 adipocytes. **e,** Differentiated 3T3-L1 adipocytes where Ampk is knocked down or pharmacologically inhibited by compound C (10 µM; f-h) show no signs of SAG-induced **f** glucose disposal, **g** lactate production or, **h** elevation in NAD⁺/NADH ratio. **i** Human Embryonic Kidney (HEK) 293 cells transfected with the AMPK-AR FRET reporter construct display a rapid and robust induction of AMPK activity in response to SAG treatment. Results are mean ± s.e.m. of at least three independent experiments performed in quadruplicate.
   * = p < 0.05 (two-tailed Student's t-test).

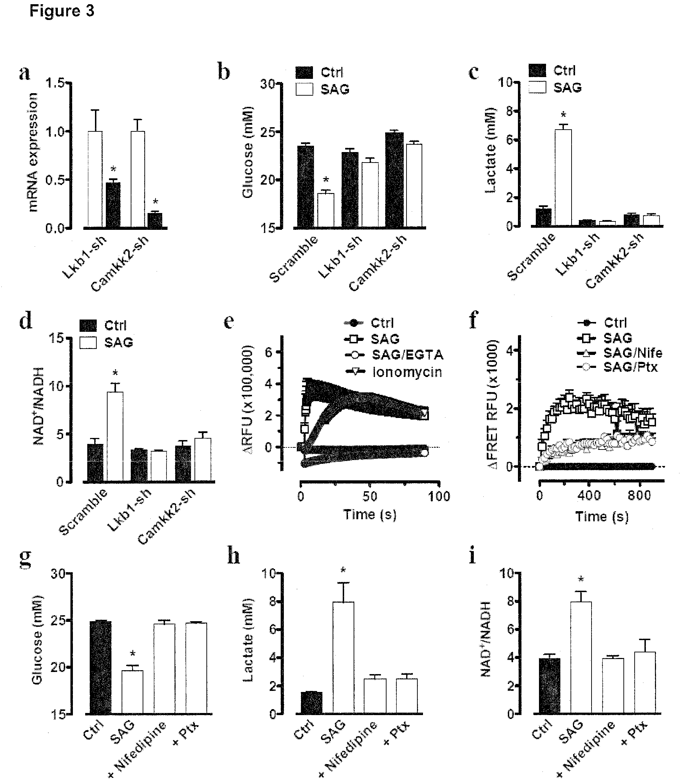

**Figure 3****: A novel Hedgehog-Ampk axis**
   **a** 3T3-L1 adipocytes where either Lkb1/Stk11 or Camkk2 are knocked down show no signs of SAG-induced **b** glucose disposal, **c** lactate production or, **d** elevation in NAD⁺/NADH ratio. **e** 3T3-L1 adipocytes were loaded with the calcium dye FluoForte-4AM and calcium response was measured. SAG-treated adipocytes show a rapid and robust calcium response, completely blunted by the extracellular calcium chelator EGTA (1 µM). **f** SAG-induced AMPK activity in HEK293 cells transfected with the AMPK-AR reporter construct is inhibited by both Nifedipine and Ptx treatment. **g** Treatment of 3T3-L1 adipocytes with the calcium channel blocker Nifedipine (20 µM) or the GPCR inhibitor Ptx (100 ng/ml) render the cells refractory to SAG-induced glucose disposal, **h** lactate production and, i elevation in NAD⁺/NADH ratio. Results are mean ± s.e.m. of at least three independent experiments performed in quadruplicate. * = p < 0.05 (two-tailed Student's t-test).

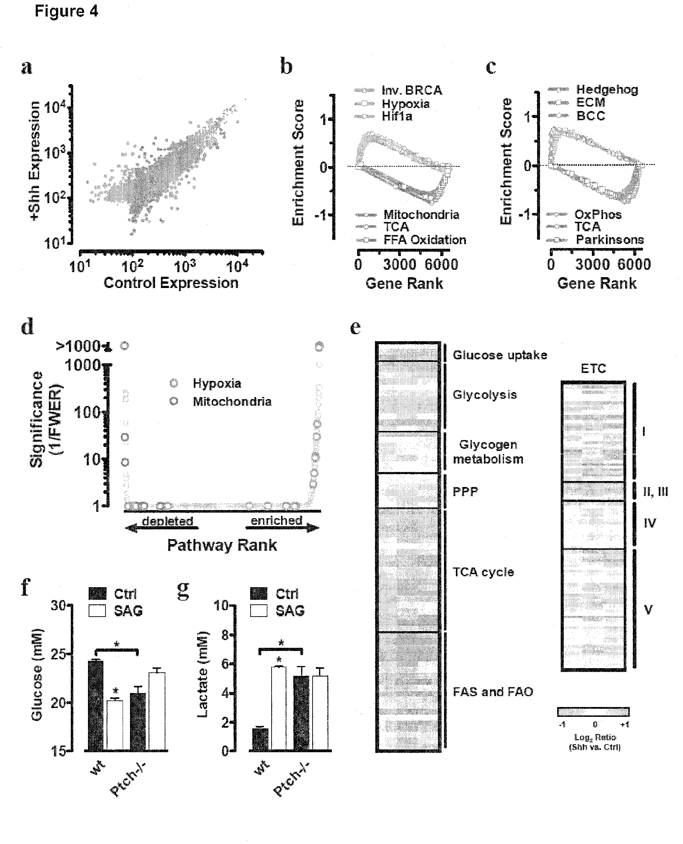

**Figure 4****: Long-term transcriptional feedback supports aerobic glycolysis.**
   **a** Microarray expression levels for 6594 expressed gene transcripts in adipocytes treated for 48 hours with or without recombinant Sonic Hedgehog (+Shh). **b-d** Gene set enrichment analysis against **b** published microarrays and **c** canonical pathways (KEGG / Biocarta) revealed striking enrichment (red) of genesets associated with hypoxia, Hedgehog, and invasive cancer, while those associated with oxidative metabolism were markedly depeleted (blue). **d** Relative to all curated genesets, hypoxia and mitochondria perturbations were over-represented at the highly significant extremes. **e** Heatmap of glycolytic and oxidative genesets highlights the switch to aerobic glycolysis. **f** MEFs deficient in the Ptch1 exhibit constitutively reprogrammed aerobic glycolytic state and are refractory to acute SAG stimulation. Results are mean ± s.e.m. of at least three independent experiments performed in quadruplicate. * = p < 0.05 (two-tailed Student's t-test).
**Figure 5****: Uncoupling of canonical and non-canonical signalling by cyclopamine reveals non-canonical signalling capable of effecting glucose uptake *in vitro* and *in vivo***
   **a** Cyclopamine (100 nM) induces metabolic reprogramming in differentiated 3T3-L1 1 adipocytes in terms of glucose consumption, **b** lactate production, and **c** elevation of NAD⁺/NADH. **d** 3T3-L1 adipocytes loaded with the calcium dye FluoForte-4AM exhibit **a** rapid Ca⁺⁺ response upon cyclopamine treamtment which is completely blunted by EGTA (1 µM). **e,** HEK293 cells transfected with the AMPK-AR reporter construct display rapid and robust induction of AMPK activity upon cyclopamine treatment, an effect blunted by both Nifedipine and Ptx. **f** Cyclopamine induced glucose consumption, **g** lactate production, and **h** elevation of NAD⁺/NADH are also abrogated by Nifedipine, Ptx and compound C treatments. **i** C57BL/6J mice display improved glucose tolerance (n = 12) when pretreated with cyclopamine (10 mg/kg, i.p.; n=12) relative to mice treated with injection vehicle alone (HBS). **j** The same mice also exhibit increase glucose-infusion rate (GIR) during glycaemic-insulinemic clamps (n = 6-10) in a dose-dependent manner (Cyc low = 1 mg/kg; Cyc high = 10 mg/kg). **k** Cyclopamine treatment (10 mg/kg) induces glucose clearance in insulin deficient STZ-treated C57BL/6J mice (n = 20). Results are mean ± s.e.m. of at least three independent experiments performed in quadruplicate. * = p < 0.05 ** = p < 0.01 *** = p < 0.001 (two-tailed Student's t-test; single-tailed t-test as for 5i.).

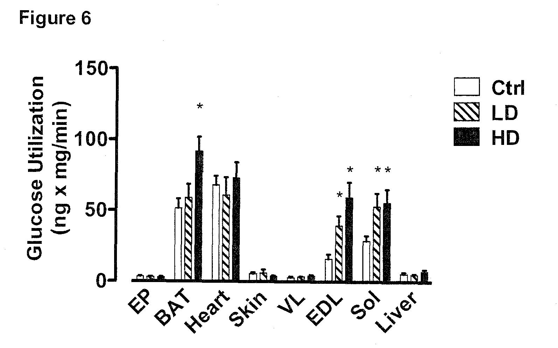

**Figure 6****: Glucose tracing shows muscle and brown adipose tissue as the primary sites of Hedgehog-induced glucose uptake in vivo**
   C57BL/6J mice display increased glucose-uptake into muscle (EDL extensor digitalis longus, Soleus, VL Vas lateralis) during glycaemic-insulinemic clamps (n = 6-10) in response to Cyclopmaine infusion (LD = 1 mg/kg; HD = 10 mg/kg). Results are mean ± s.e.m. of at least three independent experiments performed in quadruplicate. * = p < 0.05 ** (two-tailed Student's *t*-test).

The examples illustrate the invention:

### Example 1:

### Hedgehog reprograms energy metabolism

Hedgehog signalling possesses stark regulatory potential in many cellular contexts, including at least two with finely tuned metabolic programs, namely cancer and adipose tissue. Therefore, the question was whether Hedgehog signalling might determine cellular metabolic state. 3T3-L1 adipocytes were chosen as a model since they exhibit some of the most well-characterized metabolic regulatory circuits and they respond fully to Hedgehog ^{17,19,20}. Intriguingly, it was observed that stimulation of mature 3T3-L1 adipocytes with SAG *(Smoothened Agonist)²⁹,* a small molecule activator of Smo, led to acidification of culture media within 24 hours (Fig. 1a). Quantitation of aerobic / anaerobic metabolism through real-time monitoring of extracellular acidification (ECAR) and oxygen consumption (OCR) rates revealed a substantial increase in H⁺ production and reduction in O₂-consumption upon SAG stimulation (Fig. 1b), direct evidence for a shift towards aerobic glycolysis. This intriguing finding was substantiated by a marked removal of glucose from the extracellular medium and a correlate increase in lactate accumulation (Fig. 1c). Further analysis revealed elevated levels of the oxidized redox equivalents NAD⁺ and NADP⁺(Fig. 1 d), a signal that intracellular metabolite routing is shifted away from energy production and in direct agreement with the observed increase in lactate production. Importantly, levels of the reduced redox equivalents NADH and NADPH remained unchanged yielding an overall increase in redox ratios (Fig. 1 e). ATP levels also remained unaltered. These intriguing observations, concomitant with a shift towards aerobic glycolysis, are reminiscent in many ways of the Warburg effect observed in cancer, proliferative stem cells, and in activated immune cells^{3o-34}. No evidence could be found of any proliferative events in the terminally differentiated and growth arrested adipocytes. Thus, Hedgehog signalling reprograms adipocyte metabolism towards aerobic glycolysis.

To understand the kinetics of the response a time-course of glucose removal and lactate accumulation was performed in culture supernatants. Significant glucose removal and lactate generation was observed within minutes of SAG addition (Figs. 1f and 1g), a finding that is inconsistent with the kinetics of canonical transcriptionally-driven Hedgehog signalling. Importantly, pathway stimulation using the endogenous ligand Sonic hedgehog (Shh) recapitulated all aspects of the metabolic phenotype (Fig. 1h-j). Furthermore, whereas wild-type primary mouse embryonic fibroblasts (MEFs) exhibited significant SAG-stimulated shifts in glucose uptake, lactate production and even disruption of NAD'/NADH equilibrium (Fig. 1k-m), Smo^{-/-} MEFs³⁵ did not elicit such a metabolic response. Together these data provide direct genetic evidence in adipocytes and MEFs that activation of Hedgehog signalling results in a rapid and robust Smo-dependent metabolic reprogramming event. Thus, Hedgehog signalling reprograms energy metabolism.

### Example 2:

### Hedgehog triggers metabolic rewiring via Ampk

To understand how Smo activation could lead to such rapid metabolic reprogramming a phospo-proteomic survey of the acute Hedgehog signalling response was performed. Two-dimensional gel electrophoresis was used to resolve phospho-enriched lysates from differentiated 3T3-L1 adipocytes treated for 10 minutes with the Hedgehog agonist SAG (Fig. 2a). Of the 632 spots detected in three independent 2-D PAGE experiments, 55 spots comprising 39 specific phospho-proteins showed differential regulation >1.5-fold in at least 2 independent experiments. This dataset is to the inventors' knowledge the first unbiased snapshot of rapid non-canonical Hedgehog signalling reported to date and identifies numerous downstream signalling activities not previously associated with Hedgehog. Intriguingly, in a KEGG pathway analysis, all significantly enriched genesets except one constituted compartments of primary energy metabolism including *glucose, ketone body, fatty acid, pyruvate,* and ketogenic *amino acid metabolism* as well as the *TCA cycle* (Fig. 2b). Numerous key regulators of glucose and energy metabolism were identified including Acetyl-CoA acetyltransferase (Acat1), Glyceraldehyde-3-phosphate dehydrogenase (Gapdh), Transaldolase 1 (Taldo1), Hormone-sensitive lipase (Lipe), Phosphoglycerate mutase (Pgam1), Glycogen synthase (Gys), and Pyruvate dehydrogenase alpha 1 (Pdha1; Fig. 2c) indicating a complex metabolic signalling network exists downstream of Smoothened. The only non-metabolic pathway enriched in the analysis was the *proteasome* a well-known and integral component of canonical Hedgehog signalling^{5,8,12}. Thus, Hedgehog rapidly signals to the machinery of primary energy metabolism.

Considering the observed Hedgehog-driven metabolic phenotype, Western blot time-course analysis of Pdha1, Amp-activated protein kinase (Ampk) and Pyruvate kinase M1/M2 (Pkm2) phosphorylation events was performed. All three proteins have key roles in deciding glycolytic substrate fate and have been implicated in the establishment of the Warburg effect and in the regulation of cancer cell growth^{30-32,36,37}. Intriguingly, robust phosphorylation signals were observed from all three proteins, Pkm2, Pdha1, and Ampk (Fig. 2d), within two minutes of SAG stimulation, post-translational modifications all consistent with enhanced glycolysis and diversion of substrate towards a lactate endproduct.

The question was whether Ampk, as a 'master regulator' of anabolic-to-catabolic transitions^{38,39}, could be responsible for the observed metabolic phenotype, using independent genetic and pharmacological means to interfere with its activity. shRNA-based knockdown of Ampk in 3T3-L1 adipocytes to ∼20% of normal expression (Fig. 2e) completely abolished SAG-stimulated disruption of redox balance and extracellular glucose/lactate levels (Fig. 2f-h). Similarly, pre-incubation of cells with compound C, an inhibitor of Ampk activity, abrogated all detectable metabolic rewiring effects following SAG stimulation (Fig. 2f-h). Thus, Ampk activity is necessary for Hedgehog-induced metabolic rewiring.

To really define the kinetics of Hedgehog-induced Ampk activation in the system AMPKAR, a Foerster resonance energy transfer (FRET) probe for AMPK activity⁴⁰ was used. AMPKAR comprises an N- and C-terminal CFP and YFP joined by a linker peptide containing an AMPK target sequence whose phosphorylation causes protein refolding and permits FRET. Intriguingly, addition of SAG to AMPKAR expressing HEK293 cells revealed rapid and sustained AMPK-FRET signals detectable within 1 minute of stimulation (Fig. 2i). These findings show that Hedgehog stimulation results in very rapid activation of AMPK and demonstrate conservation of this novel non-canonical signalling arm in human cells, and across multiple cell types.

### Example 3:

### A novel Hedgehog-Ampk signalling axis

While predominantly studied as an activator of Gli-dependent transcriptional regulation over hours or days, Smo is a Class F G-protein coupled receptor^{41,42}, a class of proteins that effect rapid G-protein coupled second messenger signals. Ampk is principally stimulated by an increased AMP/ATP ratio, and by the upstream kinases Serine/threonine-protein kinase 11 (Stk11/Lkb1) and Calcium/calmodulin-dependent protein kinase kinase 2 (Camkk2)^{38,39}. To test for AMP input, GC/MS was used to measure AMP, ADP and ATP levels upon SAG stimulation of adipocytes. No marked dysregulation was observed (Supplementary Fig. 3a), indicating that the cells maintain normal AMP to ATP balance. Next, Lkb1 and Camkk2 was tested. Intriguingly, knockdown of either Lkb1 or Camkk2 (Fig. 3a) resulted in near-complete abrogation of downstream metabolic sequelae in response to SAG stimulation (Fig. 3b-d), suggesting that both pathways couple Smo activation to Ampk. In line with a key role for Camkk2, SAG stimulation of adipocytes preloaded with the Ca⁺⁺-sensitive fluorophore Fluoforte revealed a Ca⁺⁺ response more rapid and intense even than a standard 1 µM ionomycin stimulation (Fig. 3e). The same was true for recombinant Shh, the endogenous ligand. Administration of the extracellular calcium chelator EGTA under the same conditions completely abrogated the SAG-and Shh-induced responses, indicating that Smo activation promotes opening of plasma membrane Ca⁺⁺ channels rather than mobilizing intracellular calcium stores (Fig. 3e). Importantly, the calcium channel blocker Nifedipine markedly blunted both Ampk activity (Fig. 3f) and the key readouts of the observed metabolic phenotype, glucose uptake (Fig. 3g), lactate production (Fig.3h) and redox rebalance (Fig.3i). Moreover, Pertussis toxin (Ptx), a potent inhibitor of receptor-to-Gαᵢ G-protein coupling, abrogated Ampk activation and metabolic rerouting (Fig. 3f-i). Together, these findings indicate that Smo activation requires intact Ca⁺⁺, Camkk2, Gαᵢ, and Lkb1 coupling for full activation of the non-canonical Ampk arm. Thus, Hedgehog activation initiates rapid metabolic reprogramming through a novel Smo - Lkb1/Camkk2 - Ampk signalling axis.

### Example 4:

### Hedgehog induces long-term transcription metabolic state rewiring

Next, it was tested whether longer-term transcriptional consequences of Hedgehog signalling might in anyway complement the metabolic response. Towards this end expression profiling of adipocytes treated for 48 hours with or without the endogenous ligand Shh was performed. Analysis showed >1.5-fold up-regulation of 557 (8.5%) and down-regulation of 635 (9.6%) of 6594 expressed gene transcripts (Fig. 4a). Gene set enrichment analysis (GSEA) of the data was performed against published microarray datasets (C2:cgp, chemical and genetic perturbations) as well as canonical pathways curated from the KEGG and Biocarta databases (C2:cp) (Fig. 4b-d)⁴³. Analysis against published datasets revealed highly significant enrichment scores for gene sets associated with invasive cancer, cell migration, reduced oxidative phosphorylation, hypoxia, and the hallmark hypoxia transcriptional regulator Hif1a (Fig. 4b). Intriguingly, canonical pathways analysis revealed the primary source of these clustered signatures as Hh-induced down-regulation of primary energy metabolism including *oxidative phosphorylation, ketogenic amino acid metabolism, pyruvate metabolism, TCA cycle,* and several mitochondrial dependent neurodegenerative disorders (Fig. 4c-4e). Upregulated signals originated predominantly from cell-cell interaction, extra cellular matrix, cancer, and of course, Hedgehog signalling itself. Together, these transcriptional data indicate that Hedgehog signalling drives a long-term transcriptional gene signature that functionally parallels the observed acute metabolic response. The data are in keeping with the observation that Ptch-^{/-} cells, which display constitutively active canonical Hedgehog signalling⁴⁴, exhibit high glucose utilization and lactate production while being refractory to an acute SAG-induced metabolic shift (Fig. 4f, g). Thus, Hedgehog signalling initiates biphasic metabolic rewiring, including a second, long-term transcriptional phase.

### Example 5:

### Partial agonism of Ampk-dependent metabolic rewiring in vitro and in vivo

Currently numerous Hedgehog inhibitors are under clinical development in Phase I and Phase II trials for their potential in blocking cancer growth¹³. In an attempt to tease apart the relative contributions of canonical Gli-dependent and non-canonical Ampk-dependent signalling the inventors began to test well-characterized Hedgehog antagonists for their potential to block the identified rapid metabolic response. Surprisingly, when the classic Hedgehog antagonist cyclopamine ^{44,45} was added to the 3T3-L1 cells (initially as a negative control), rapid and robust metabolic reprogramming was observed as determined by marked glucose uptake (Fig. 5a), generation of lactate (Fig. 5b), and augmentation of the NAD⁺/NADH ratio (Fig. 5c). Of note, these effects were observed even at modest concentrations (100 nM) where cyclopamine has been reported to bind efficiently to Smo but is incapable of displacing standard 200 nM SAG and blocking canonical signalling^{29,45,46}. This unique finding shows that Smo-modulators can activate rapid non-canonical signalling independently of the canonical arm and identifies cyclopamine as a potent partial agonist of the pathway. Importantly, like SAG, cyclopamine induces a rapid and robust Ca⁺⁺ response sensitive to extracellular EGTA (Fig. 5d). It prompts a significant Ampk sensitive FRET signal, which, again like SAG, is sensitive to the calcium channel blocker Nifedipine, and the G-protein toxin Ptx (Fig. 5e), evidence that both SAG and cyclopamine stimulate the same non-canonical signalling machinery. For completion, cyclopamine-dependent glucose uptake, lactate production, and redox rebalancing were also all efficiently counteracted by addition of either Nifedipine, Ptx or the Ampk inhibitor compound C (Fig. 5f-h). These data clearly show that Ampk- and Gli-dependent signalling arms can be chemically uncoupled at the level of Smo and thus give unique new insight into the fundamental mechanism of Hedgehog signalling activation.

Perhaps equally important, these finding provided us with a tool to probe the relevance of the non-canonical axis *in vivo.* To test whether Smo partial agonism could boost metabolism oral glucose tolerance tests (OGTT) was performed on wild-type C57BL/6J mice in the presence or absence of acute cyclopamine pre-treatment. Impressively, mice receiving 10 mg/kg cyclopamine 15 minutes prior to testing displayed a moderate but significant improvement in glucose tolerance (Fig. 5i). Neither basal nor glucose-stimulated insulin secretion appeared affected by the pre-treatment. Since insulin is known to counteract Ampk aCtivity⁴⁷, it was reasoned that an acute insulin response might be masking the full potential of the pathway. To bypass this gold standard glycemic-insulinemic clamps were used using continuous somatostatin infusion to suppress acute insulin secretion⁴⁸. Impressively, under these conditions wild-type C57BL/6J mice exhibited robust dose-dependent increases in glucose infusion rate within 10-20 minutes of cyclopamine infusion (Fig. 5j). These findings provide direct evidence that acute Smo-dependent metabolic rewiring occurs *in vivo.* Thus, Smo partial agonism induces glucose uptake.

Finally, the latter two findings raised the intriguing question whether Smo-induced glucose clearance might be effective even in the complete absence of insulin. To test this hypothesis, cyclopamine tolerance tests were performed in STZ-treated mice. STZ is a toxin that specifically kills pancraeatic beta-cells and renders animals type-1 diabetic and devoid of insulin secretory function. Remarkably, despite a complete lack of circulating insulin, STZ-treated mice showed significant and robust glucose clearance within ∼30 minutes of cyclopamine injection (Fig. 5k), a remarkable stimulation of insulin independent glucose uptake *in* vivo.

Glucose tracer experiments tracking tissue-specific glucose uptake during the clamp studies interestingly showed that the majority of elevated glucose administration was routed in vivo to muscle (EDL and Sol; extensor digitorum longus and soleus) as well as brown adipose tissue (BAT) indicating that Smo modulation is capable of activating Brown adipose tissue metabolism (Figure 6).

These data demonstrate Smo-dependent metabolic rewiring for the first time *in vivo,* and identify Smo partial agonism as a novel therapeutic strategy for the treatment of insulin-dependent metabolic disorders such as Type-1 and late stage Type-2 diabetes mellitus.

### Example 6: Material and Methods

### 6.1 Cell culture, transfection and viral infection

Murine 3T3-L1 preadipocytes (ATCC) were maintained in DMEM containing 10% heat inactivated calf serum (CS) at 37°C and 5% CO₂. Two days post-confluence, adipocyte differentiation was induced by treating cells with 0.25 mM 3-Isobutyl-1-methylxanthine (IBMX), 1 µM dexamethasone, 1.74 µM insulin and 5 µM Troglitazone in DMEM containing 10% fetal bovine serum (FBS). After 48 hours, the medium was replaced with DMEM supplemented with 10% FBS, insulin and Troglitazone alone. After an additional 48 hours, cells were switched to DMEM with 10% FBS for the rest of the differentiation process and the medium was changed every other day. Seven days post-induction, differentiated 3T3-L1 1 adipocytes were starved overnight in DMEM containing 1 % Bovine Serum Albumin (BSA) before starting different treatments as indicated in the text. For treatments the following compounds and concentrations were used: 200 nM SAG (Alexis Biochemicals), 0.25 µg/ml recombinant Shh (R&D Systems), 100 nM cyclopamine, 10 µM compound C, 20 µM nifedipine and 100 ng/ml pertussis toxin (Ptx; Calbiochem).

Primary mouse embryonic fibroblasts (MEFs) were grown in DMEM with 10% FBS at 37°C and 5% CO₂. MEFs lacking functional Ptch (Ptch^{-/-}) were derived from Ptch^{-/-} mouse embryos as described previously²⁰. *Smo^{-l-}* MEFs were kindly provided by Jussi Taipale³⁵. For experiments, MEFs were grown to confluence and, 2 days post-confluence, were starved in DMEM with 1 % BSA for 16 hours. The next day, treatments were performed as indicated in the text.

For lentiviral transduction, preadipocytes were seeded overnight in 12-well plates (Corning) in DMEM containing 10% CS. The following morning 5 MOl of lentivirus were complexed with 5 mg/ml Polybrene (Santa Cruz Biotechnology), added to the cells and left 24 hours before being removed and replaced with DMEM containing 10% CS. Puromycin was used to select stable clones. Transduction ready lentiviruses expressing shRNAs targeting Ampka1 (RMM3981-9591411), Lkb1/Stk11 (RMM3981-9591552) and Camkk2 (RMM3981-9593143) were obtained from OpenBiosystems. A lentivirus harboring a scrambled shRNA was used as control (OpenBiosystems, RHS4459).

HEK293 cells (ATCC) were grown in DMEM containing 10% FBS. 2 x 10⁶ cells were seeded in 10 cm dishes and transfected using the GeneJuice Reagent (Merck) according to manufacturer's instructions. 48 hours after transfection, cells were resuspended in PBS and used for FRET assays.

### 6.2 Cellular bioenergetics

Analysis of bioenergetic function was performed using a Seahorse XF24 Extracellular Flux Analyzer (Seahorse Bioscience). Briefly, differentiated 3T3-L1 adipocytes were seeded into 24 well Seahorse V7 plates. Adherent cells were pretreated with either vehicle or SAG (200 nM) for 6 hours. Pretreated cells were transferred to the Flux Analyzer to record real-time oxygen consumption rates (OCR) as well as extracellular acidification rates (ECAR). The measurement protocol consisted of 4 minutes mixture, 2 minutes wait and 2 minutes OCR/ECAR measurement times. Cellular DNA was measured using CyQuant (Invitrogen) to adjust for potential differences in cell densities.

### 6.3 Metabolite determination

Differentiated 3T3-L1 adipocytes were starved overnight in DMEM containing 1 % BSA and treated as indicated. NAD⁺, NADP⁺, NADH, and NADPH were determined using the Enzychrom NAD⁺/NADH and the Enzychrom NADP⁺/NADPH assay kit, respectively (BioAssay Systems) according to manufacturer's instructions. To measure glucose consumption and lactate production, the Glucose RTU and Lactate RTU kits (Biomerieux) were used according to manufacturer's instruction and normalized to cell number. AMP, ADP and ATP were determined as previously described⁵¹. In brief, cells were washed once with ice cold PBS and proteins were precipitated with 1 % trichloroacetic acid (TCA). Repeated ether washes were used to remove TCA from the aqueous extract phase. Extracts were dried and stored at -80°C until further processing. To determine nucleotide phosphates by reversed-phase HPLC cell extracts were lyophilized for 3 days at -20 °C. Dried samples were dissolved in mobile phase A (60 mM K₂HP0₄ and 40 mM KH₂P0₄, adjusted to pH 7 with 0.1 M KOH). 20 µL were injected into an Agilent 1200 HPLC system (Agilent) and nucleotides were separated by gradient elution from an Agilent Eclipse XDB-C18 4.6 x 150 mm column at a flow rate of 1 mL/min. The gradient was 99% phase A from 0-5 minutes, 99% phase A to 40% A from 5-20 minutes and 99% phase A from 20-32.5 minutes. 100% acetonitrile served as mobile phase B. Eluting nucleotides were monitored on a UV detector at 254 nm. ATP, ADP and AMP standards were used to generate calibration curves for peak quantifications.

### 6.4 Phospho-proteomics and 2D-PAGE

Phosphoprotein enrichment was performed by affinity column purification of total adipocyte protein using a PhosphoProtein Purification Kit (Qiagen) according to the manufacture's instructions. Briefly, ∼10⁷ cells were washed twice with ice cold HEPES buffer on ice, resuspended in 5 mL lysis buffer containing protease and phosphatase inhibitors and Benzonase nuclease, provided by the manufacturer. The cell suspension was incubated on ice for 30-60 minutes with vortexing every 10 minutes. Subsequently, cell lysates were centrifuged at 10.000 g and 4°C for 30 minutes. PhosphoProtein purification columns were equilibrated with lysis buffer and 2.5 mg protein loaded on the column. Columns were washed once with lysis buffer and phosphoproteins were eluted five times with the elution buffer. Eluted fractions were combined, concentrated and desalted using Vivaspin™ sample concentrators (GE Healthcare) with a 10 kDa molecular weight cut-off according to the instructions of the manufacturer. Protein concentration was determined using the Bradford or BCA (Pierce) method for proteins diluted in sample buffer (7 M Urea, 2 M Thiourea, 2% CHAPS, 1 % Amberlite) supplemented with nuclease mix (Amersham Biosciences), protease (GE Healthcare) and phosphatase inhibitors (Roche) or RIPA buffer (50 mM Tris-HClpH 7.6, 150 mM NaCl, 1 mM EDTA, 1% Triton X-100, 1% Sodium Deoxycholate, 0,1% SDS), respectively.

For 2D-PAGE, immobilized pH gradient (IPG) strips (pH 3-10, 17cm) were passively rehydrated with 50-100 µg protein in 280 µL. Isoelectric focusing (IEF) was performed on an Ettan lPGphor II system (GE Healthcare). Focused IPG strips were equilibrated in equilibration buffer (6 M Urea, 50 mM Tris pH 8.8, 2% SDS, 20% glycerol) with 100 mM 1,4-dithio-D,L-threitol (DTT) and 2.5% iodacetamide (lAA) for 10-15 minutes. Equilibrated IPG strips were placed on top of 12% acrylamide/1,4-Bis (acryloyl) piperazine (AA/PDA) gels and overlaid with 0.5% low-melting agarose. Proteins were separated by SDS-PAGE using an Ettan DALTtwelve (GE Healthcare) at 5 W per gel for 30 minutes followed by 15 W per gel until the dye front completely ran off. The gels were then stained with a solution of Ruthenium II tris (bathophenanthroline disulfonate) (RuBPS). Briefly, gels were fixed overnight in 50% methanol / 10% acetic acid, washed twice with 20% methanol for 20 minutes, stained with a 400 nM solution of RUBPS in 20% methanol for 6-8 hours and destained in 50% methanol / 7% acetic acid overnight. Stained gels were scanned with a Fluorlmager 595 (GE Healthcare) at a resolution of 100 µm.All 2D-gel data were independently produced three times. Detection, matching, background subtraction, normalization and quantitative comparison of 2D spots were accomplished using the Progenesis SameSpots software (Nonlinear Dynamics).

### 6.5 Silver staining and in-gel digest

After washing, 2D-gels were sensitized in 0.02% Na₂S₂0₃, stained with ice-cold 0.1 % AgN0₃ and developed with 3% Na₂CO₃/0.5% formaldehyde. Spots of interest were excised manually and stored in 1% acetic acid until further processing. The gel-pieces were destained (150 mM potassium hexacyanoferrate, 500 mM sodium thiosulfate) and washed with 50% methanol / 10% acetic acid over night. The pH was neutralized with 50 mM NH₄HCO₃, proteins were reduced with 10 mM DTT in 50 mM NH₄HCO₃ at 56°C for 30 minutes and alkylated with 50 mM iodacetamide in 50 mM NH₄HCO₃ in the dark for 20 minutes. Subsequently, gel-pieces were treated with acetonitrile and dried in a vacuum centrifuge. Between each step, the tubes were shaken at room temperature for 5-10 minutes. Dry gelspots were digested with 0.1 mg/ml trypsin (Roche) in 50 mM NH₄HCO₃ on ice for 30 minutes and subsequently stored at 37°C over night. Digested peptides were eluted by adding 5% formic acid / 50% acetonitrile. The supernatant was transferred into silicon-coated tubes and the elution procedure was repeated two times. Between each elution step the gel-pieces were ultrasonicated at 160 W for 10 minutes. Finally, the peptide solution was concentrated in a vacuum centrifuge to a total volume of approximately 20 µL.

### 6.6 LC/MSMS analysis

For mass spectrometry based protein identification, tryptic digests were loaded on a Zorbax 300SB-C8 column (5 µm, 0.3 mm, 5 mm) using the HPLC-Chip technology (Agilent) and separated by nanoflow LC (1100 Series LC system, Agilent) with a Zorbax 300SB-C18 column (5 µm, 75 mm, 150 mm) at a flow rate of 250 nL/min using a gradient from 0.2% formic acid and 3% acetonitrile to 0.2% formic acid and 45% acetonitrile over 12 minutes. Peptide identification was accomplished by MSMS fragmentation analysis with an iontrap mass spectrometer (XCT-Ultra, Agilent) equipped with an orthogonal nanospray ion source. The MSMS data, including peak list generation and search engine, were interpreted by the Spectrum Mill MS Proteomics Workbench software (Version A.03.03, Agilent) allowing for two missed cleavages and searched against the SwissProt Database for murine proteins (Version 14.3 containing 20.328 entries) allowing for precursor mass deviation of 1.5 Da, a product mass tolerance of 0.7 Da and a minimum matched peak intensity (% Scored Peak Intensity) of 70%. Carbamidomethylation of cysteines was set as fixed modification with no other modifications considered. To assess the reliability of the peptide scores, searches against the corresponding reverse database were performed. 6.0% positive hits were found with peptides scoring >9.0, while 0.2% positive hits were found with peptides scoring >13.0. Consequently, the threshold for protein identification was set scoring 14.0 or higher, resulting in a false discovery rate of 0.1 %.

Data were further processed and quantified with the Scaffold software tool (Version 3.0, Proteome Software). Protein identifications were accepted if they contained at least two identified peptides. Proteins that contained similar peptides and could not be differentiated based on MSMS analysis alone were manually verified.

### 6.7 Western blotting

Cells were washed twice with ice cold PBS on ice and lysed in RIPA buffer (50 mM Tris-HCl, pH 7.6, 150 mM NaCl, 1 mM EDTA, 1 % Triton X-100, 1 % Sodium Deoxycholate, 0.1% SDS) containing protease and phosphatase inhibitors (Roche). Cell lysates were cleared by centrifugation at 4°C at 16000 g for 30 minutes and protein concentration in the supernatant was determined by BCA (Pierce). 20 µg of proteins were resolved by SDS-PAGE and transferred to PVDF membranes (GE Healthcare). Membranes were blocked with 5% BSA in Tris-buffered saline containing 0.2% Tween-20 (TBS-T), and incubated with primary antibodies at 4°C over night. The following antibodies were used (all from Cell Signaling unless indicated otherwise): anti-phospho-AMPK (pThr172) (1:5.000; Cat.-Nr. 2535), anti-total AMPK (1:5.000; Cat.-Nr. 2532), anti-phospho-PKM2 (pTyr105) (1:5.000; Cat.-Nr. 3827), anti-total PKM2 (1:10.000; Cat.-Nr. 3198), anti-phospho-PDHA1 (1:50.000; ab77844, Abcam), anti-total PDHA1 (1:5.000; Cat.-Nr. 3205), and HRP-linked anti-rabbit IgG (1:5.000; Cat.-Nr. 7074). Antigen-specific binding of antibodies was detected with SuperSignal West Femto and Pico Kits (Pierce) using a ChemiDoc XRS Imager (Bio-Rad). Image analysis was performed using Image Lab Software Version 3.0.1. (Bio-Rad).

### 6.8 AMPKAR / Foerster resonance energy transfer (FRET) assay

HEK293 cells transfected with the AMPK-AR construct⁴⁰ were resuspended in PBS at a concentration of 10⁵ cells/ml. A Synergy 4 microplate reader (BioTeK) was used for measuring FRET signals. In brief, compounds were pipetted into a 96-well plate at the concentrations indicated in the text. Cell suspensions were pre-loaded in the reader to be injected at 10⁴ cells/well. The FRET signal was detected using 420/50 nm and 528/20 nm as excitation and emission settings. Baseline values for each well were acquired before injection and subtracted afterwards.

### 6.9 Calcium flux measurement

3T3-L1 cells were cultured and differentiated in black wall/clear bottom 96-well plates (Corning). At day seven post-induction, cells were starved overnight in DMEM containing 1 % BSA and loaded with the calcium dye FluoForte AM (Enzo Lifescience) according to the instructions provided by the manufacturer. Intracellular calcium levels were measured using a Synergy 4 microplate reader (BioTeK). Briefly, compounds were injected at the reported concentration and calcium flux was measured by detecting fluorescence at 488/530 nm (excitation/emission). Baselines for each well were acquired before injection and subtracted afterwards. Ionomycin and EGTA were injected in concentrations of 1 µM.

### 6.10 Gene Expression Analysis

Total RNA from 3T3-L1 adipocytes was isolated using the RNeasy Mini Kit (Qiagen) according to the manufacturer's protocol. The integrity and quantity of RNA was analyzed using the Agilent Bioanalyzer (Agilent Technologies). Preparation of complementary RNA (cRNA), hybridization to the GeneChip Mouse Gene 1.0 ST Array (Affymetrix), and scanning of arrays were conducted according to the manufacturer's protocols (http://www.affymetrix.com). Robust multi-array average (RMA) signal extraction and normalization filtering was performed. Filtering criteria included normalized expression level of >100 in at least one sample. Filtered genes were subjected to Gene Set Enrichment Analysis (GSEA) available for free at the Broad Institute (http://www.broadinstitute.org/gsea/index.jsp)⁴³.

### 6.11 1 Quantitative RT-PCR

Total RNA was extracted from cells using TRlzol according to the manufacturer's instructions. Isolated RNA was reverse-transcribed (RT) into cDNA using iScript cDNA Synthesis Kit (Bio-Rad). Quantitative RT-PCR was performed using iQ SYBR Green Supermix (Bio-Rad) on an AbiPRISM 7500fast real-time cycler (Applied Biosystems). Post-amplification melting curve analysis was performed to check for unspecific products and primer-only controls were included to ensure the absence of primer dimers. Threshold cycles (Cₜ-values) of all replicate analyses were normalized to hypoxanthine-guanine phosphoribosyltransferase (*Hprt*). To compare the effect of various treatments with untreated controls, 2^{-ΔΔCt} values were calculated to obtain fold expression levels. Primer sequences used are listed in Supplementary Table 6.

### 6.12 Mouse studies

C57BL/6J mice were maintained on a standard rodent chow diet and a 12 hour light: 12 hour dark cycle. Eight week old, over-night fasted C57BL/6J male mice were injected intraperitoneally (i.p.) with vehicle (45% hydroxypropyl-β-cyclodextrin; HBS) or cyclopamine (10 mg/kg). Fifteen minutes after injection, an oral glucose tolerance test (OGTT) was performed by administering 2 g/kg glucose by oral gavage. Tail-vein blood was collected to measure glucose and insulin levels. Insulin was measured using the ultrasensitive insulin ELISA Kit from Mercodia, according to the manufacturer's recommendations. For cyclopamine tolerance tests eight week old C57BL/6J mice were i.p. injected with streptozotocin (STZ, 200 mg/kg body weight) to destroy pancreatic beta-cells. 72 hours later STZ-treated mice were separated into two groups and injected i.p. with either vehicle or 10 mg/kg cyclopamine. All animal experiments were carried out according to an ethical animal license protocol in accordance to the animal protection law and approved by the Regierungspräsidium Freiburg (Tierversuchsvorhaben Registrier-Nr.: G-10/94).

### 6.13 Glycemic-insulinemic clamp studies

Indwelling catheters were placed into the left femoral vein and externalised in the interscapular region of 11 week old C57BL/6J mice^{17,52}. The animals were allowed to recover for 5 days and fasted for 16 hours before the experiment. Somatostatin (SST14 Bachem, Switzerland) was infused at a rate of 1 µg/kg/min and hyperglycemia (12.5 mM) was maintained by variable infusion of 10% glucose. Steady state was maintained for 90 min before cyclopamine injection (2/3 bolus 1/3 infusion; low dose = 1 mg/kg, high-dose = 10 mg/kg), or HBS (Control). Whole blood was sampled from the tail every 10 min during the last hour using a glucometer (AccuCheck Aviva; Roche Diagnostics, Meylan, France).

### 6.14 Statistical Analysis

All data unless otherwise indicated are shown as mean values ± standard error of the mean (s.e.m.). Data sets were compared for statistical significance using two-tailed Student's t-tests for unpaired samples. All figures and statistical analyses were generated using GraphPad Prism 5.0. p < 0.05 was considered to indicate statistical significance.

### References

1. Ingham, P. W. & McMahon, A. P. Hedgehog signaling in animal development: paradigms and principles. Genes Dev. 15, 3059-3087 (2001).
2. Rohatgi, R. & Scott, M. P. Patching the gaps in Hedgehog signalling. Nat. Cell Biol. 9, 1005-1009 (2007).
3. Ingham, P. W., Nakano, Y. & Seger, C. Mechanisms and functions of Hedgehog signalling across the metazoa. Nat. Rev. Genet. 12, 393-406 (2011).
4. Ruiz i Altaba, A. Hedgehog Signaling and the Gli Code in Stem Cells, Cancer, and Metastases. Sci. Signal. 4, pt9 (2011).
5. Hui, C.-c. Gli Proteins in Development and Disease. Annu. Rev. Cell Dev. Biol. 27, 513-537 (2010).
6. Varjosalo, M. & Taipale, J. Hedgehog: functions and mechanisms. Genes Dev. 22, 2454-2472 (2008).
7. Riobo, N. A. & Manning, D. R. Pathways of signal transduction employed by vertebrate Hedgehogs. Biochem. J. 403, 369 (2007).
8. Lauth, M. & Toftgård R. Non-canonical activation of GLI transcription factors: implications for targeted anti-cancer therapy. Cell cycle 6, 2458-2463 (2007).
9. Chinchilla, P., Xiao, L., Kazanietz, M. G. & Riobo, N. A. Hedgehog proteins activate pro-angiogenic responses in endothelial cells through non-canonical signaling pathways. Cell cycle 9, 570-579 (2010).
10. Yam, P. T., Langlois, S. D., Morin, S. & Charron, F. Sonic Hedgehog Guides Axons through a Noncanonical, Src-Family-Kinase-Dependent Signaling Pathway. Neuron 62, 349-362 (2009).
11. Jenkins, D. Hedgehog signalling: Emerging evidence for non-canonical pathways. Cell. Signal. 21, 1023-1034 (2009).
12. Teglund, S. & Toftgård, R. Hedgehog beyond medulloblastoma and basal cell carcinoma. Biochim. Biophys. Acta 1805, 181-208 (2010).
13. Ng, J. M. Y. & Curran, T. The Hedgehog's tale: developing strategies for targeting cancer. Nat. Rev. Cancer 11, 493-501 (2011).
14. Berman, D. M. Medulloblastoma Growth Inhibition by Hedgehog Pathway Blockade. Science 297, 1559-1561 (2002).
15. Berman, D. M. et al. Widespread requirement for Hedgehog ligand stimulation in growth of digestive tract tumours. Nature 425, 846-851 (2003).
16. Thayer, S. P. et al. Hedgehog is an early and late mediator of pancreatic cancer tumorigenesis. Nature 425, 851-856 (2003).
17. Pospisilik, J. A. et al. Drosophila Genome-wide Obesity Screen Reveals Hedgehog as a Determinant of Brown versus White Adipose Cell Fate. Cell 140, 148-160 (2010).
18. Xu, Z., Yu, S., Hsu, C.-H., Eguchi, J. & Rosen, E. D. The orphan nuclear receptor chicken ovalbumin upstream promoter-transcription factor II is a critical regulator of adipogenesis. Proc. Natl. Acad. Sci. U. S. A. 105, 2421-2426 (2008).
19. Suh, J. M. et al. Hedgehog signaling plays a conserved role in inhibiting fat formation. Cell Metab. 3, 25-34 (2006).
20. Todoric, J. et al. Cross-Talk Between Interferon- and Hedgehog Signaling Regulates Adipogenesis. Diabetes 60, 1668-1676 (2011).
21. Lowe, C. E., O'Rahilly, S. & Rochford, J. J. Adipogenesis at a glance. J. Cell Sci. 124, 2681-2686 (2011).
22. Lumeng, C. N. & Saltiel, A. R. Inflammatory links between obesity and metabolic disease. J. Clin. Invest. 121, 2111-2117 (2011).
23. Arner, P. et al. Dynamics of human adipose lipid turnover in health and metabolic disease. Nature 478, 110-113 (2011).
24. Whittle, A. J., López, M. & Vidal-Puig, A. Using brown adipose tissue to treat obesity - the central issue. Trends Mol. Med. 8, 405-411 (2011).
25. Yauch, R. L. et al. A paracrine requirement for hedgehog signalling in cancer. Nature 455, 406-410 (2008).
26. Chen, W. et al. Canonical hedgehog signaling augments tumor angiogenesis by induction of VEGF-A in stromal perivascular cells. Proc. Natl. Acad. Sci. U. S. A. 108, 9589-9594 (2011).
27. Theunissen, J. W. & de Sauvage, F. J. Paracrine Hedgehog signaling in cancer. Cancer Res. 69, 6007-6010 (2009).
28. Bijlsma, M. F. & Roelink, H. Non-cell-autonomous signaling by Shh in tumors: challenges and opportunities for therapeutic targets. Expert Opin. Ther. Targets 14, 693-702 (2010).
29. Chen, J. K., Taipale, J., Young, K. E., Maiti, T. & Beachy, P. A. Small molecule modulation of Smoothened activity. Proc. Natl. Acad. Sci. U. S. A. 99, 14071-14076 (2002).
30. Vander Heiden, M. G., Cantley, L. C. & Thompson, C. B. Understanding the Warburg effect: the metabolic requirements of cell proliferation. Science 324, 1029-1033 (2009).
31. Hsu, P. P. & Sabatini, D. M. Cancer Cell Metabolism: Warburg and Beyond. Cell 134, 703-707 (2008).
32. Dang, C. V., Hamaker, M., Sun, P., Le, A. & Gao, P. Therapeutic targeting of cancer cell metabolism. J. Mol. Med. 89, 205-212 (2011).
33. Shi, L. Z. et al. HlF1 -dependent glycolytic pathway orchestrates a metabolic checkpoint for the differentiation of TH17 and Treg cells. J. Exp. Med. 208, 1367-1376 (2011).
34. Fox, C. J., Hammerman, P. S. & Thompson, C. B. Fuel feeds function: energy metabolism and the T-cell response. Nat. Rev. Immunol. 5, 844-852 (2005).
35. Varjosalo, M., Li, S.-P. & Taipale, J. Divergence of Hedgehog Signal Transduction Mechanism between Drosophila and Mammals. Dev. Cell 10, 177-186 (2006).
36. Hitosugi, T. et al. Tyrosine Phosphorylation of Mitochondrial Pyruvate Dehydrogenase Kinase 1 Is Important for Cancer Metabolism. Mol. Cell 44, 864-877 (2011).
37. Kim, J. W. & Dang, C. V. Cancer's molecular sweet tooth and the Warburg effect. Cancer Res. 66, 8927-8930 (2006).
38. Canto, C. & Auwerx, J. Calorie Restriction: Is AMPK a Key Sensor and Effector? Physiology 26, 214-224 (2011).
39. Hardie, D. G. AMP-activated protein kinase--an energy sensor that regulates all aspects of cell function. Genes Dev. 25, 1895-1908 (2011).
40. Tsou, P., Zheng, B., Hsu, C.-H., Sasaki, A. T. & Cantley, L. C. A Fluorescent Reporter of AMPK Activity and Cellular Energy Stress. Cell Metab. 13, 476-486 (2011).
41. Ogden, S. K. et al. G protein Gαi functions immediately downstream of Smoothened in Hedgehog signalling. Nature 456, 967-970 (2008).
42. Polizio, A. H., Chinchilla, P., Chen, X., Manning, D. R. & Riobo, N. A. Sonic Hedgehog Activates the GTPases Rac1 and RhoA in a Gli-independent Manner Through Coupling of Smoothened to Gi Proteins. Sci. Signal. 4, pt7 (2011).
43. Subramanian, A. et al. Gene set enrichment analysis: a knowledge-based approach for interpreting genome-wide expression profiles. Proc. Natl. Acad. Sci. U. S. A. 102, 15545-15550 (2005).
44. Taipale, J. et al. Effects of oncogenic mutations in Smoothened and Patched can be reversed by cyclopamine. Nature 406, 1005-1009 (2000).
45. Chen, J. K., Taipale, J., Cooper, M. K. & Beachy, P. A. Inhibition of Hedgehog signaling by direct binding of cyclopamine to Smoothened. Genes Dev. 16, 2743-2748 (2002).
46. Stanton, B. Z. & Peng, L. F. Small-molecule modulators of the Sonic Hedgehog signaling pathway. Mol. Biosyst. 6, 44-54 (2010).
47. Kovacic, S. et al. Akt activity negatively regulates phosphorylation of AMP-activated protein kinase in the heart. J. Biol. Chem. 278, 39422-39427 (2003).
48. Krebs, M. et al. Mechanism of amino acid-induced skeletal muscle insulin resistance in humans. Diabetes 51, 599-605 (2002).
49. Lavine, K. J., Kovacs, A. & Ornitz, D. M. Hedgehog signaling is critical for maintenance of the adult coronary vasculature in mice. J. Clin. Invest. (2008).
50. Pola, R. et al. The morphogen Sonic hedgehog is an indirect angiogenic agent upregulating two families of angiogenic growth factors. Nat. Med. 7, 706-711 (2001).

## Claims

1. A modulator of the Smoothened (Smo) receptor capable of altering cellular glucose uptake for use in the treatment of a disease, wherein
(a) the modulator is an activator of the Smoothened (Smo) receptor capable of increasing cellular glucose uptake and is for use in the treatment of diabetes, glucose intolerance, hyperglycemia, obesity, overweight, sepsis and diseases associated therewith; or
(b) wherein the modulator is an inhibitor of the Smoothened (Smo) receptor capable of decreasing cellular glucose uptake and is for use in the treatment of hypoglycaemia.

2. The modulator of claim 1, wherein the diabetes is selected from type 1 diabetes, type 2 diabetes, type 3 diabetes, maturity onset diabetes of the young (MODY), neonatal diabetes, diabetes type 1.5, steroid-induced diabetes, pre-diabetes, gestational diabetes, type A insulin resistance, leprechaunism, Rabson-Mendenhall syndrome, lipoatrophic diabetes; diabetes associated with Down syndrome, Klinefelter syndrome, Turner syndrome, Wolfraram syndrome, Friedreich ataxia, Huntington chorea, Laurence-Moon-Biedl syndrome, myotonic dystrophy, porphyria and Prader-Willi syndrome; diabetes caused by pancreatitis, trauma/pancreatectomy, neoplasia, cystic fibrosis, hemochromatosis, and/or fibrocalculous pancreatopathy; diabetes caused by acromegaly, Cushing's syndrome, glucagonoma, pheochromocytoma, hyperthyroidism, somatostatinoma, and/or aldosteronoma; and diabetes that is induced by vacor, pentamidine, nicotinic acid, glucocorticoids, thyroid hormone, diazoxide, β-adrenergic agonists, thiazides, dilantin and/or γ-lnterferon; diabetes cause by congenital rubella and/or cytomegalovirus infection.

3. The modulator of claim 1 or 2, wherein the diseases associated with diabetes are selected from the group consisting of obesity, hyperlipidemia, hypercholesterolemia, diabetic coma, diabetic ketoacidosis, hyperglycemia, diabetic cardiomyopathy, diabetic nephropathy, diabetic neuropathy, diabetic retinopathy, diabetic coronary artery disease, diabetic myonecrosis, peripheral vascular disease, and stroke.

4. A method of altering the glucose uptake, the lactate level, the NAD⁺/NADH ratio and/or the NADP⁺/NADPH ratio of a cell, said method comprising the step of (a) contacting said cell with a modulator of the Smoothened (Smo) receptor capable of altering glucose uptake, lactate level, NAD⁺/NADH ratio and/or NADP⁺/NADPH ratio, respectively.

5. The method for altering the glucose uptake of claim 4, further comprising a step (b) of contacting the cell of step (a) with glucose or a glucose analogue labelled with a detectable moiety prior to, simultaneously with or after having contacted said cell of step (a) with said modulator of the Smoothened (Smo) receptor capable of altering glucose uptake.

6. A method of identifying a modulator of the Smoothened (Smo) receptor capable of altering the glucose uptake, the lactate level, the NAD⁺/NADH ratio and/or the NADP⁺/NADPH ratio of a cell, comprising the steps of:
(a) determining the level of glucose uptake, the level of lactate, the NAD⁺/NADH ratio and/or the NADP⁺/NADPH ratio in a cell containing Smo receptors;
(b) contacting said cell or a cell of the same cell population with a test compound;
(c) determining the level of glucose uptake, the level of lactate, the NAD⁺/NADH ratio and/or the NADP⁺/NADPH ratio in said cell after contacting with the test compound;
(d) comparing the level of glucose uptake, the level of lactate, the NAD⁺/NADH ratio and/or the NADP⁺/NADPH ratio determined in step (c) with the glucose uptake level, the lactate level, the NAD⁺/NADH ratio and/or the NADP⁺/NADPH ratio determined in step (a); and
(e) determining prior to, simultaneously with or after any one of the preceding steps (a) to (d) whether said test agent modulates the Smo receptor, wherein a change of the glucose uptake level, a change of the lactate level, a change of the NAD⁺/NADH ratio and/or a change of the NADP⁺/NADPH ratio in step (c) as compared to step (a) and modulation of the Smo receptor by the test compound indicates that said test compound is a modulator of the Smo receptor capable of altering the glucose uptake, the lactate level, the NAD⁺/NADH ratio and/or the NADP⁺/NADPH ratio of a cell.

7. The method of claim 6, wherein step (e) comprises:
(a) assessing whether said test compound binds to the Smo receptor or a cellular activator of the Smo receptor, wherein binding of said test compound to said Smo receptor or said cellular activator of the Smo receptor indicates that said test compound modulates the Smo receptor; and/or
(b) determining the level of Smo receptor protein or Smo receptor transcript in a cell according to claim 6(a);
(c) determining the level of Smo receptor protein or Smo receptor transcript in said cell according to claim 6(b) after contact with said test compound; and
(d) comparing the level of Smo receptor protein or Smo receptor transcript determined in step (c) with the Smo receptor protein or Smo receptor transcript level determined in step (a), wherein a change of Smo receptor protein or Smo receptor transcript level in step (c) as compared to step (a) indicates that the test compound modulates the Smo receptor.

8. The modulator of claim 1 or the method of any one of claims 2 to 7, wherein the modulator or the test compound is an antibody or a fragment or derivative thereof, an aptamer, an siRNA, an shRNA, a miRNA, a ribozyme, an antisense nucleic acid molecule, a small molecule or modified versions of these modulators or test compounds.

9. A method of increasing uptake of glucose or a glucose analogue labelled with a detectable moiety into a cell, said method comprising the steps of:
(a) contacting said cell with an activator of the Smoothened (Smo) receptor capable of increasing glucose uptake, and
(b) contacting the cell of step (a) with glucose or a glucose analogue labelled with a detectable moiety prior to, simultaneously with or after having contacted said cell of step (a) with said activator of the Smoothened (Smo) receptor capable of increasing glucose uptake.

10. The method of claim 5 or claim 9, wherein the detectable moiety is selected from the group consisting of a radionuclide, a luminophore or an enzyme.

11. The method of claim 10, wherein the glucose or glucose analogue labelled with a radionuclide is suitable for use as an imaging agent in positron emission tomography (PET).

12. The modulator of any one of claims 1 to 3 or 8, or the method of any one of claims 4 to 11, wherein the modulator or activator binds to the Smo receptor or an activator of the Smo receptor.

13. The modulator or method of claim 12, wherein the activator or the modulator, in case it is an activator of the Smo receptor, is selected from the group consisting of SAG, cyclopamine, purmorphamine, SANT-1, SANT-2, SANT-3, SANT-4, GDC-0449, NVP-LDE225, NVP-LEQ506, IPI609, lPl-926, BMS-833923, PF-04449913, TAK-441, Cur-61414, LY2940680, Itraconazol, or Vitamin D.

14. The modulator of any one of claims 1 to 3, 8, 12 or 13 or the method of any one of claims 4 to 13, wherein the activator or modulator, in case it is an activator of the Smo receptor, is further capable of activating G-proteins via the Smo-receptor, phosphorylating Amp-activated protein kinase (Ampk) and opening Ca²⁺-channels.
